# NEW EUROPEAN PATENT SPECIFICATION

(11) **EP 0 454 784 B2**
(45) Date of publication and mention of the opposition decision: **16.01.2008**
(45) Mention of the grant of the patent: 17.04.1996
(21) Application number: 90902964.7
(22) Date of filing: 16.01.1990
(51) Int. Cl.: C12Q 1/18

(54) **APPARATUS AND METHODS FOR ANTIMICROBIAL SUSCEPTIBILITY TESTING OF MICROORGANISMS**
VORRICHTUNG UND VERFAHREN ZUM TESTEN DER ANTIMIKROBIELLEN EMPFINDLICHKEIT VON MIKROORGANISMEN
APPAREIL ET PROCEDES POUR TESTER LA SUSCEPTIBILITE ANTIMICROBIENNE DE MICRO-ORGANISMES

(30) Priority: 17.01.1989 US 298599
(43) Date of publication of application: 06.11.1991
(73) Proprietor: ALAMAR BIOSCIENCES LABORATORY INC., Sacramento, CA 95823 (US)
(72) Inventor: LANCASTER, Michael, V., Woodland, CA 95695 (US); FIELDS, Rebecca, D., Woodland, CA 95695 (US)
(74) Representative: Baldock, Sharon Claire
(86) International application number: PCT/US1990/000149
(87) International publication number: WO 1990/008196

(56) References cited:
- ABSTRACTS OF THE ANNUAL MEETING OF THE AMERICAN SOCIETY FOR MICROBIOLOGY vol. 87, 1987, WASHINGTON DC USA page 332; R.W. KELLY ET AL.: 'A fluorescent system for two hour identification of gram negative bacteria.'
- ABSTRACTS OF THE ANNUAL MEETING OF THE AMERICAN SOCIETY FOR MICROBIOLOGY vol. 90, 1990, WASHINGTON DC USA page 386; M. LANCASTER ET AL.: 'New colorimetric method for determining antibiotic minimal inhibitory concentration.'
- J.Labadie et M.Doumbia; Zbl.Bakt.Hyg.1. Abt.Orig B 179, 1984, pages 217-224
- A.Pital, Ruth C Pital, J.M Leise; Am.Rev.Pulm.Dis.(1958) vol.78, pages 111-116

## Description

### BACKGROUND OF THE INVENTION

### Field of the Invention

This invention relates generally to apparatus and methods for testing the susceptibility of a microorganism to growth inhibition by an antimicrobial product. This invention relates to both qualitative susceptibility and quantitative susceptibility testing of microorganisms.

### Prior Art in General

### Source of organisms and culturing for test purposes

Microorganism specimens to be tested may be supplied to the laboratory from a number of sources. The specimens may be collected by doctors in their offices and sent to a central testing laboratory or the specimens may be collected from patients in a hospital with which the laboratory is associated. The specimens may come from various parts of the body, for example, from cerebral spinal fluid, an abscess, an infected wound, a genital infection, etc. The collected specimens are cultured on a primary agar plate in accordance with normal laboratory practice. From the bacterial colonies on the primary culture plate an inoculum is prepared in accordance with an established procedure which produces a bacterial suspension of a prearranged concentration. Further processing of the inoculum depends on the apparatus and method to be used for susceptibility testing.

### Susceptibility Testing Apparatus and Methods

The purpose of susceptibility testing is to provide information to the referring physician on the probable success of the antibiotic drug therapy that has already been initiated. The physician will generally prescribe an antimicrobial product, commonly called an antibiotic drug, to be administered before the test results are known, but it is often important for the physician to learn whether that antimicrobial product and the concentration given will successfully kill the microorganism that is causing the infection. After the test results are in, the physician can change the drug therapy if the test results show that there is a reason to do so.

US-A-4,385,115 discloses an identification method for microorganisms in which microorganisms are grown on a substrate comprising a growth indicator, such as resazurin.

Qualitative vs. Quantitative Susceptibility Testing The term qualitative susceptibility testing refers to testing apparatus and methods which produce test results that generally indicate whether an organism is sensistive or resistant to a particular antimicrobial product. Depending on the method involved only one or two concentrations of antimicrobial product are utilized. The degree of sensitivity or resistance is not reported in qualitative susceptibility testing.

The term quantitative susceptibility testing refers to testing apparatus and methods which produce test results that provide data on the concentration of the antimicrobial product that will be sufficient to inhibit growth of the microorganism. Typically six or more different dilutions of the antimicrobial product are utilized covering the therapeutic range of concentrations of the antimicrobial product. The term Minimum Inhibitory Concentration (MIC) is often used to refer to the result provided by quantitative susceptibility testing and is defined as the minimum concentration of the antimicrobial product which will produce inhibition of the growth of the microorganism.

The term antimicrobial product will be used herein to designate a product that contains a set of antimicrobial agents (i.e. individual antibiotics) in prearranged concentrations and is thus a general designation for a single antibiotic drug or a broad spectrum formulation that contains more than one antibiotic agent.

### Qualitative Susceptibility Testing Apparatus and Methods

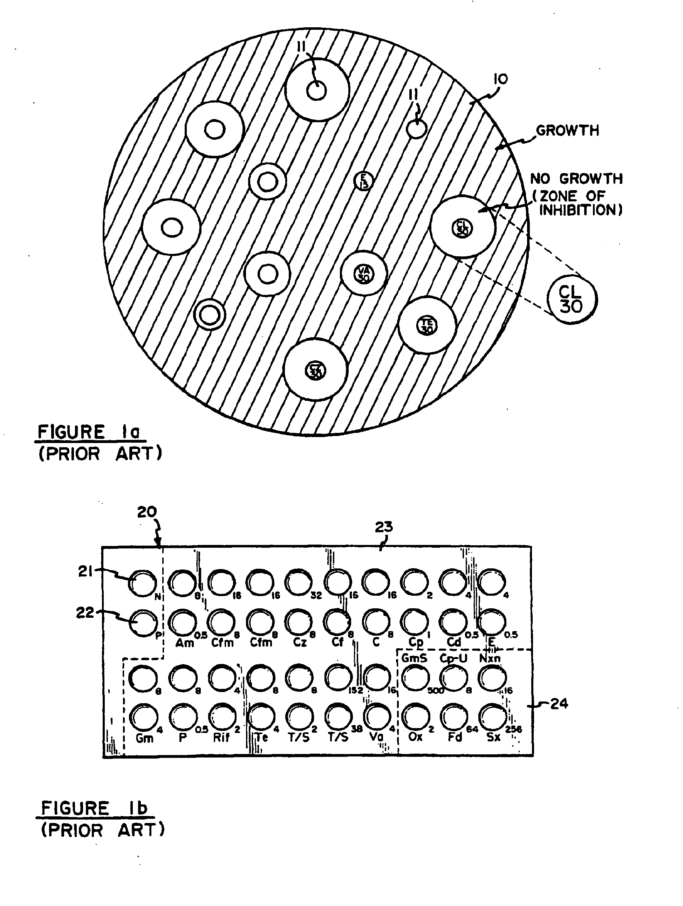

### Disk Diffusion on Kirby/Bauer Plates (Fig. 1A)

Disk diffusion is a method of qualitative susceptibility testing which utilizes a plate 10 covered with a growth medium for the microorganism, typically called a Kirby/Bauer plate, an inoculum broth, and a plurality of paper disks 11 on which an antimicrobial product has been stored. The plate may be one hundred and fifty millimeters in diameter, and the disks six millimeters in diameter. The inoculum broth containing the microorganism is coated over the plate forming a uniform lawn of the organism. The paper disks are then dropped onto the plate at separated locations and the plate is then placed in an incubator to allow growth of the microorganism Fig. 1A illustrates an example of the appearance of the plate after incubation. The zone of inhibition surrounding the disks may vary from zero to twenty five or thirty millimeters.

During incubation, the antimicrobial product in the disk will diffuse into the microorganism lawn and form a continuous distribution of different concentrations of the antimicrobial product. Growth of the microorganism will typically be inhibited in the central region of the diffused zone where the concentration of antimicrobial product is greatest unless the microorganism is highly resistant to the antimicrobial product. At some radius from the center of the disk growth of the microorganism will be seen. The size of the zone of inhibition around a particular disk gives an indication of the susceptibility of the microorganism to the particular antimicrobial product thereon.

Fig. 1A and Table I (found at the end of this specification) illustrate a typical example of some of the antimicrobial products used and the results of reading a disk diffusion test.

The principal advantages of the disk diffusion method are flexibility in the selection of antimicrobial products for testing and ease of set up of the test. Disk storage and dispenser systems have been developed for simultaneously dispensing a plurality of disks containing different antimicrobial products onto the plate at appropriately separated locations. The dispenser system can be loaded with different disk storage modules, giving the user complete flexibility in choosing the antimicrobial products to use in each specimen testing situation.

The principal disadvantage is that the "sensitive" and "resistant" interpretation of the test results are based on attainable serum levels of antibiotic which may not correlate well to attainable antibiotic levels of the infection site other than blood. This is true of all qualitative susceptibility testing methods. Other disadvantages are the difficult and time consuming process of reading and interpreting the test, using a ruler to measure zone size and refering to a chart for interpretation. Another is that difference in interpretation of "Sensitive" and "Resistant" is often only a few millimeters, so errors can occur due to variations in test methodology resulting from heavy inoculum or other variations. The disk diffusion methodology also limits the number of antimicrobial products that may be tested on one plate.

### Breakpoint Test Panels (Fig. 1B)

Another approach to qualitative susceptibility testing involves the use of multiwell test panel system that are called breakpoint test panel systems or Kirby-Bauer test panel systems. As shown in Fig. 1B breakpoint test panels systems typically utilize a multiwell test panel 20 with a negative growth control well 21, a positive growth control well 22, a first panel section 23 in which two different dilutions of multiple antimicrobial products are used and a second panel section 24 in which only one dilution of each of several antimicrobial products is present. The test wells of the panel are labelled with an abbreviation of the associated antimicrobial product and the concentration in the well is given in micrograms per milliliter. Table II at the end of the specification gives the antimicrobial products and breakpoint concentrations used in this example of a prior art breakpoint panel.

The negative growth control well 21 contains only growth medium and is not inoculated with the microorganism. It is principally used to monitor the panel for contamination. If organism growth in the positive growth control well is detected after incubation, then the panel is rejected and the test must be repeated. The positive growth control well 22 contains growth medium but no antimicrobial product and is inoculated with the microorganism. The positive growth control well serves the purpose of demonstrating that the growth medium in the panel is capable of growing the microorganism outside of the presence of an antimicrobial product. If there is no growth of the microorganism in the positive growth control well, the test is invalid and must be repeated on another test panel.

Each of the test wells associated with the antimicrobial products contains growth medium and one known concentration of the associated antimicrobial product. These test chemicals are deposited into the wells and then either frozen or dried. Frozen panels are thawed just prior to inoculation. Dried panels are rehydrated at the time of the inoculation with the microorganism using separate rehydration of the negative growth control well with a sterile rehydrating solution.

The two different concentrations of each antimicrobial product in panel section 23 are selected to provide information on whether a particular microorganism is sensitive or resistant to that antimicrobial product. The concentrations are based on correlation of the test results with the disk diffusion test methods and results described above.

Each of the positive growth control well and the wells associated with each antimicrobial product are inoculated with a prearranged uniform concentration of the microorganism. The panel is then incubated, typically overnight, and then visually read. If the negative growth control well and positive growth control well show that the panel qualifies for reading, then each of the pairs of test wells in panel section 23 or the single test well in panel section 24 associated with the antimicrobial products is inspected to determine which wells show growth of the microorganism.

Referring to the two dilution panel section 23, if the microorganism is growing in each of the two wells, the test result for that antimicrobial product is "resistant." If the microorganism is not growing in either well, the test result is "sensitive." If the organism is growing in the lower concentration well and not growing in the higher concentration well, the test result is "indeterminant." If the organism were growing in the higher concentration well and not in the lower concentration well, this would indicate a failed test.

In the single dilution panel section 24, sensitive and resistant are called as test results depending on growth or no growth in the single well. If there is growth, the microorganism is resistant at that level of antimicrobial product; and if there is no growth, the microorganism is sensitive at that level.

The principal advantage of qualitative susceptibility test panels over disk diffusion testing and quantitative susceptibility test panels discussed below is that a larger number of antimicrobial products can be used in the test panel since only one or two dilutions per antimicrobial product are required. Another advantage is that the panel is relatively easier and faster to read than disk diffusion test results.

The principal disadvantage of breakpoint test panels is the same as that discussed above for disk diffusion testing. In addition, currently available qualitative susceptibility test panels provide no flexibility in testing the specimen with user selected antimicrobial products unless the user purchases sufficient quantities such that the manufacturer will be willing to make a custom panel. Generally, the manufacturer has preselected the antimicrobial products to put on the panel and the user takes the panel as it is supplied. The manufacturer may remove a drug for which the user has a substantial testing demand. There is usually a time delay in putting newly available drugs on the commercial test panels. In both of these cases, the user must make other testing arrangements if testing of the microorganism against these antimicrobial products is required.

### Quantitative Susceptibility Testing

### Manual Reading Systems

Most of the quantitative susceptibility testing done in clinical laboratories today uses multiwell test panels with multiple dilutions of from twelve to twenty three different antimicrobial products along with a negative growth control well and a positive growth control well. Most of the reading of such panels is done manually by visual reading of the panel. Visual reading involves looking for turbidity in the test well as an indicator of organism growth. All of the test wells for each antimicrobial product are inspected and the well with the lowest concentration of antimicrobial product in which turbidity is not present is the MIC.

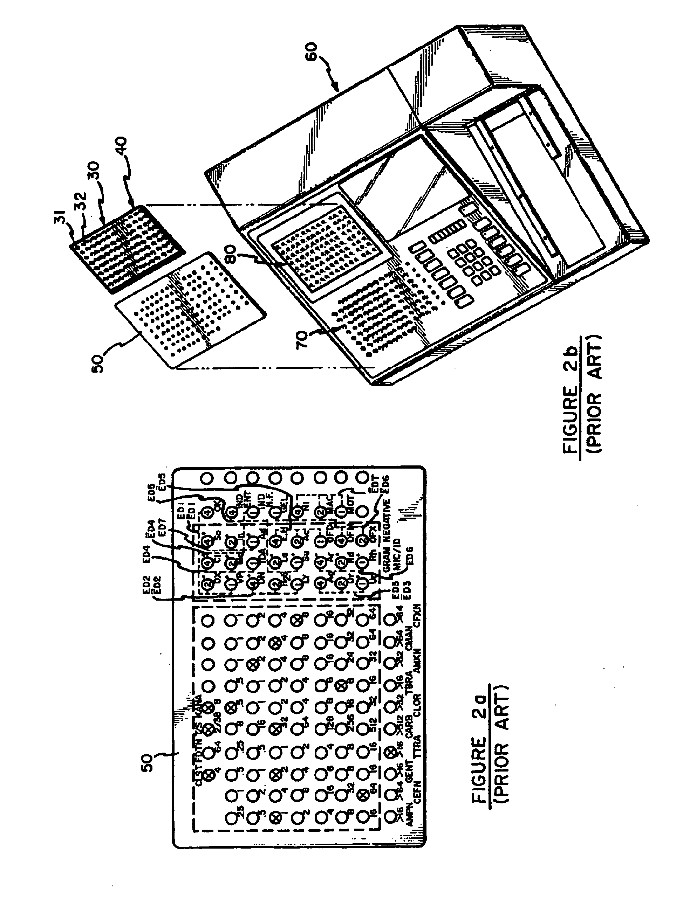

Figs. 2A and 2B illustrate a test panel reading data recording system which is covered by Lancaster et al U.S. Patent 4,453,220. This overall system is typical of the visual reading and computer based datalogging systems of the prior art. The antimicrobial products and dilutions shown in Fig. 2A are typical of the MIC test panel technology at the time the'220 patent was filed. Different antimicrobial products and dilutions may be used in current MIC test panel technology.

Figs. 2A and 2B illustrate that a MIC test panel 30 may be combined with an identification panel 40 for the microorganism. A keyboard overlay 50 shown in Fig. 2A corresponds with test well pattern in the MIC panel 30 and the ID panel 40 and fits over the keyboard section 70 of the data entry system 60. The panels 30 and 40 fit into a backlighted reader section 80 which illuminates the test wells from the back to make it easier to determine if turbidity is present in the test wells. While reading the MIC panel 30, the technologist presses the key on keyboard section 70 corresponding to the well with the lowest concentration of the associated antimicrobial product in which the microorganism is not growing. This is the MIC value for that antimicrobial product. The instrument 60 records this MIC value for that antimicrobial product and later prints it on a test report form. Further details of this manual reader and data entry and printing system can be found in the above-referenced patent.

The primary advantage of quantitative susceptibility test panels is the quantitative information provided by the test results. The actual concentration of antimicrobial product that inhibits growth of the microorganism is determined and can be used by the physician to select an appropriate antimicrobial product and dosage for the involved infection or to confirm an initial drug therapy already initiated.

The principal disadvantages of MIC test panels are the limited number of antimicrobial products and concentrations that are available for testing on the panel and the fixed selection of the antimicrobial products by the manufacturer. While a large user can order custom panels with selected antimicrobial products, most users have no control over the antimicrobial products on the panel.

An additional disadvantage is the difficulty often encountered in determining whether there is growth or not in a particular test well. Sometimes the amount of turbidity is small and difficult to see and in other cases the organism growth pattern may make it difficult to detect growth.

### Automated Reading Systems

Automated reading systems are available for determining growth or no growth of the microorganism in quantitative susceptibility test panels. Reading is based either on detecting turbidity in the test wells of the panel or by detecting the production of specific enzymes by the microorganism using specific fluorescent substrates.

There are two basic types of automated reading systems: those that involve overnight incubation of the test panel prior to reading and those that involve reading after four to six hours of incubation. The latter are commonly referred to as rapid systems. The automated reader systems for test panels requiring overnight incubation use turbidimetric reading with some form of optical measurement using through transmission of light. Thus all require a clear test panel and clear media in the test wells of the panel. A consistent optical path requires high quality plastic and control of test well liquid in terms of volume and consistency of optical parameters.

In this type of automated reading, the test panel can be read visually as well as automatically.

The rapid automated reading systems use either turbidimetric reading or fluorescent reading. The test panels involved in these systems cannot be read visually as a back up to the automated reading or to confirm the results if the automated system is suspected of malfunctioning.

The only advantage of the automated readers for overnight test panels is the labor saving in reading and reporting the data from the test panel. The test results are not more accurate than that produced by visual reading by a skilled person. The mai disadvantage of these reading systems is the cost of the system and the possibility of machine errors in reading and interpreting difficult growth patterns of some microorganisms.

The advantage of rapid automated reading systems is the labor saving in reading and reporting the data and the earlier reporting of test results. As noted above, a disadvantage of these systems is that the test panels cannot be read visually as a backup to machine reading.

### Objects of the Invention

It is the principal object of this invention is to provide improved apparatus and methods for antimicrobial susceptibility testing of microorganisms.

It is another object of this invention to provide apparatus and methods for antimicrobial susceptibility testing of microorganisms with improved ease of reading of the test panel.

It is another object of this invention to provide apparatus and methods for antimicrobial susceptibility testing of microorganisms with improved colorimetric and fluorescence reading of the test panels.

It is another object of this invention to provide apparatus and methods for qualitative susceptibility testing of microorganisms with improved flexibility of selection of antimicrobial products.

It is another object of this invention to provide apparatus and methods for quantitative susceptibility testing of microorganisms with improved flexibility of selection of antimicrobial products.

It is another object of this invention to provide apparatus and methods for susceptibility testing of microorganisms wherein the test panels can be read manually or automatically.

### Features and Advantages of the Invention

One aspect of this invention features a method for testing the susceptibility of a microorganism to growth inhibition by a preselected concentration of an antimicrobial product comprising the steps of :
(a) disposing in each of a negative growth control recepticle and a positive growth control recepticle a prearranged concentration of a growth medium for microorganisms and a prearranged concentration of resazurin predetermined to be in a concentration range characterised by low toxicity to microorganisms and substantial sensitivity to reduction to resorufin by metabolic products of microorganism growth, and a pre-selected redox stabiliser characterised by substantial lowering of the reduction of resazurin by said growth medium during step (g);
(b) disposing in said positive growth control recepticle a prearranged concentration of said microorganism;
(c) disposing in a test recepticle said preselected concentration of said antimicrobial product;
(d) disposing in said test recepticle said prearranged concentration of resazurin, and a preselected redox stabiliser characterised by substantial lowering of the reduction of resazurin by said growth medium during step g;
(e) disposing in said test recepticle said prearranged concentration of growth medium ;
(f) disposing in said test recepticle said prearranged concentration of said microorganism;
(g) incubating all of said recepticles together for an incubating time period associated with a preselected reading protocol comprising one of a visible light reading protocol and a fluorescence excitation reading protocol; and
(h) after said incubating time period, reading said recepticles in accordance with said preselected reading protocol to determine the presence or absence of growth of said microorganism in said test receptible on the basis of the relative concentrations of resazurin and resorufin therein, said visible light reading including a decision algorithm based on at least one predetermined functional combination of the visible light reflectantance color detected in each of said recepticles, said fluorescence excitation reading protocol including a decision algorithm based on at least one predetermined functional combination of the values of the fluorescence emission signal produced by the reduction product resorufin in each of said recepticles.

One of the advantages of the method of this invention is the availability of the two different reading protocols from the same testing methodology. Both the visible light reading protocol and the fluorescence excitation reading protocol can be automated and the latter can be utilized in a rapid testing protocol to determined growth or no growth in a four to six hour period of incubating time. Another advantage is that visual reading in accordance with the visible light reading protocol is greatly facilitated by the use of color change from the original blue color of resazurin toward the red color of resorufin when the microorganism has grown in the test well. In side by side comparisons with visual reading of turbidity, it has been shown that reading growth with color change is faster and easier.

In one embodiment of the method of this invention, the steps of preparing the test recepticle comprise first forming in the test recepticle a test module comprising a dry solid volume of a prearranged quantity of the antimicrobial product and a dry solid volume of a preselected subset of the constituents of a set of test chemicals comprising resazurin and the growth medium; and then dispensing into the test well a volume of liquid having therein the prearranged concentration of the microorganism and all of the constituents of the set of test chemicals not in the test module to rehydrate the test module prior incubation. Preferably the set of test chemicals includes a preselected buffer for controlling the final pH of the rehydrated test chemical solution. A preselected redox stabilizer characterized by substantial lowering of the reduction of resazurin by the growth medium during the incubation step is included in the set of test chemicals.

It is also preferable to use a carrying medium such as an absorbent paper disk in the recepticles with the test chemicals in each recepticle dried into the carrying medium. The method of this invention provides flexibility in placing various components of the set of test chemicals in the carrying medium or the rehydrating liquid, but it is generally preferred that all of the test chemical components be placed into the carrying medium so that the rehydrating liquid need carry only the microorganism to be placed in the positive growth control well and the test well.

The method of this invention advantageously permits the use of standard growth media in the test chemical set and the use of the carrying medium provides flexibility in selection of the antimicrobial products to be used when the method of the invention is used in connection with qualitative susceptibility test panels or quantitative susceptibility test panels. Such panels can be fully preloaded panels and provide the ease of reading advantage of this invention. The panels can be fully loadable by using the dried carrying medium, preferably in the form of absorbent paper disks with the antimicrobial product and concentration printed thereon, to give full flexibility to the user in selection of antimicrobial products and concentrations. In addition, test panels with a combination of preloaded test modules and bare test wells for loading user selected test modules can be provided.

Test kits with disk dispensers and other test components can be provided in accordance with this invention to make loading disks into the test wells a simple process.

Other objects, features, and advantages of this invention will be apparent from a consideration of the detailed description given below in conjunction with the accompanying drawing figures.

### Brief Description of the Drawings

Fig. 1A illustrates the results of a disk diffusion test on a Kirby-Bauer plate in accordance with the prior art.

Fig. 1B illustrates a qualitative susceptibility test panel in accordance with the prior art.

Fig. 2A and 2B illustrate a quantitative susceptibility test panel and a manual reading and data entry system in accordance with the prior art.

Fig. 3 illustrates the general method and apparatus for determining the susceptibility of a microorganism to growth inhibition by an antimicrobial product in accordance with this invention.

Figs. 4-8 illustrate a visible light reading protocol in accordance with this invention.

Figs. 9-12 illustrate various alternative embodiments of method and apparatus for determining the susceptibility of a microorganism to growth inhibition by an antimicrobial product in accordance with this invention.

Fig. 13 illustrates use of the method and apparatus of this invention in a qualitative susceptibility test panel.

Figs. 14-16 illustrate a visible light reading protocol for the qualitative susceptibility test panel of Fig. 13.

Fig. 17 illustrates use of the method and apparatus of this invention in a quantitative susceptibility test panel.

Figs. 18 and 19 are illustrative examples of a visible light reading protocol for the quantitative susceptibility test panel of Fig. 17.

Figs. 20A and 20B illustrate components of alternative embodiments of qualitative susceptibility test kits in accordance with this invention.

Fig. 21 illustrates a qualitative susceptibility test panel in accordance with this invention with preloaded test modules for multiple antimicrobial products.

Fig. 22 illustrates a qualitative -susceptibility test panel in accordance with this invention with test wells loadable with user selected test modules for multiple antimicrobial products.

Fig. 23 illustrates a qualitative susceptibility test panel in accordance with this invention with both preloaded and bare test wells for multiple antimicrobial products.

Figs. 24A and 24B illustrate components of alternative embodiments of quantitative susceptibility test kits in accordance with this invention.

Fig. 25 illustrates an alternative set of test kit components for loading test modules into test wells of a bare test panel for quantitative susceptibility testing.

Fig. 26 illustrates a quantitative susceptibility test panel in accordance with this invention having preloaded test modules for multiple antimicrobial products.

Fig. 27 illustrates a quantitative susceptibility test panel in accordance with this invention having bare test wells for loading with user selected test modules for multiple antimicrobial products.

Fig. 28 illustrates test kit components for loading test modules for multiple antimicrobial products into bare test wells of a test panel in accordance with this invention.

Fig. 29 illustrates a quantitative susceptibility test panel in accordance with this invention having a section of preloaded test modules for multiple preselected antimicrobial products and a section of bare test wells for loading of test modules for multiple user selected antimicrobial products.

Fig. 30 is a schematic illustration of an automated panel reader system useful in accordance with this invention.

Fig. 31 illustrates a visible light reading system useful in connection with a visible light reading protocol for a test panel in accordance with this invention.

Fig. 32 illustrates a fluorescence excitation reading system useful in connection with a fluorescence excitation reading protocol for a test panel in accordance with this invention.

Fig. 33 is a graph showing the results of fluorescence reading of microorganism growth in a test well in accordance with the method of this invention compared with other prior art reading methods.

Fig. 34 is a graph that illustrates the fluorescence reading of microorganism growth in a test well in accordance with the method of this invention with different microorganisms.

### Detailed Description of Embodiments of the Invention

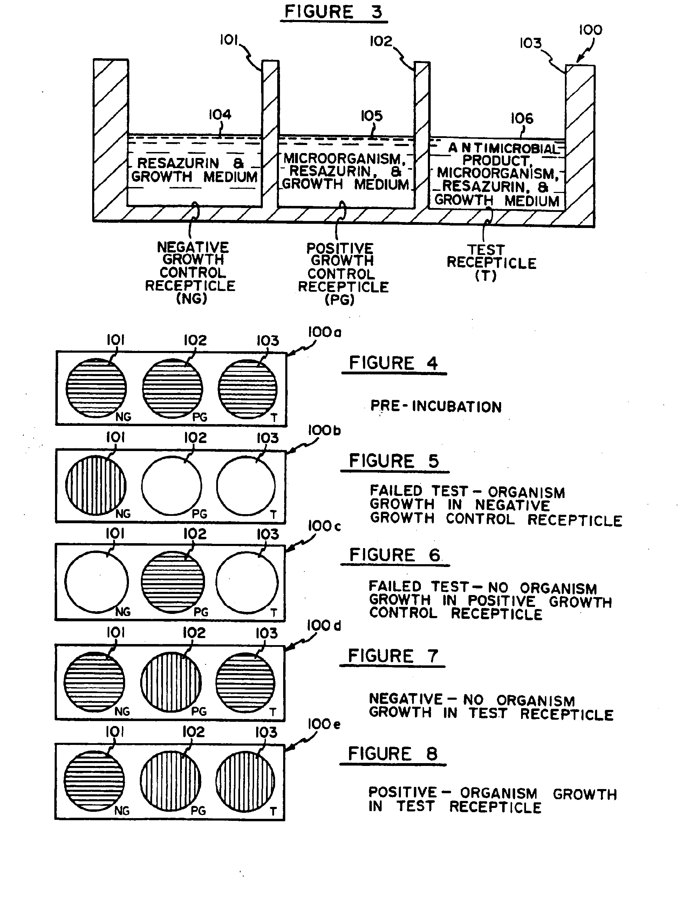

### The Basic Methodology (Figs. 3-8)

The basic method of this invention involves testing the susceptibility of a microorganism to growth inhibition by a preselected concentration of an antimicrobial product utilizing a test panel 100 with a negative growth control well or recepticle 101, a positive growth control well or recepticle 102, and a test well or test recepticle 103. The term "well" or "recepticle" will be used interchangably in this description with the understanding that the term "recepticle" is general to any appropriate structure that for holding the test chemicals. The method is not dependent upon use of a multiwell panel, and separate individual recepticles could be used. The panel approach is preferred for simplicity of handling in and out of the incubator and for other reasons that are well know to persons in this art. The general steps of the method will now be described.

A prearranged concentration of a growth medium for microorganisms is disposed in the two growth control recepticles 101 and 102, a prearranged concentration of resazurin is also disposed in both growth control recepticles and a preselected redox stabilises characterised by substantial lowering of the reduction of resazurin by said growth medium during step (g). The growth medium may, for example, be standard Mueller-Hinton broth and the concentration of this growth medium may be in the standard range of concentrations currently used in the susceptibility testing industry. The concentration of resazurin used is in a predetermined range characterized by low toxicity to microorganisms and substantial sensitivity to reduction to resorufin by the metabolic products of microorganism growth.

A prearranged concentration of the microorganism to be tested is disposed in the positive growth control well 102. Thus, as shown in Fig 3, the negative growth control well 102 contains growth medium and resazurin while the positive growth control well contains growth medium, resazurin and the microorganism.

In the test well 103 the following test chemicals are disposed: the antimicrobial product in the preselected concentration, the same prearranged concentration of growth medium as in the two growth control wells, the same concentration of resazurin and the same concentration of microorganism as in the positive growth control well

After all of the test chemicals are disposed in the three recepticles, thcy are incubated together for an incubating time period associated with a preselected reading protocol. Generally the reading protocols which are available to use with this invention are a visible light reading protocol and a fluorescence excitation reading protocol. More details of these various reading protocols are given below.

After the incubating time period, the three recepticles are read in accordance with the preselected reading protocol to determine the presence or absence of growth of the microorganism in the test well on the basis of the relative concentrations of resazurin and resorufin therein. The visible light reading protocol includes a decision algorithm based on at least one predetermined functional combination of the visible light reflectance color detected in each of the three test wells. The fluorescence excitation reading protocol includes a decision algorithm based on at least one predetermined functional combination of the values of the fluorescence emission signal produced by the reduction product resorufin in each of the test wells. The details of the reading protocols in accordance with this invention are given below.

### The Reduction/Oxidation Reaction of Resazurin

In the method and apparatus of this invention, resazurin is used as a reduction/oxidation indicator. When microorganisms grow in a growth medium, they convert nutrients to energy, resulting in a chemical reduction of their environment. This is true for all microorganisms. If an oxidation/reduction indicator is present in the environment of the growing microorganism, it will also be reduced. Thus, the use of an oxidation/reduction indicator provides a universally applicable test for growth of all microorganisms. Resazurin is such an oxidation/reduction indicator and is reduced to resorufin. Resazurin is deep blue in reflected color and non-fluorescent. Resorufin is red and highly fluorescent. This reduction of resazurin to resorufin is the basis for the visible light reading protocol and the fluorescence excitation reading protocol utilizied in the method of this invention.

Prior art fluorescence reading systems for susceptibility testing do not incorporate a universal indicator of microorganism growth. Instead, they use fluorescent substrates which measure production of specific enzymes and there is no one fluorescent substrate that will be utilized by all microorganisms. Resazurin reduction is not an enzyme based reaction but rather a chemical reaction depending on a change in the oxidation/reduction state of the environment. It is independent of -enzymatic reaction.

Resazurin is also a pH indicator, having a blue color above a pH of about 6.5 to 6.8 and being red below that range of pH values. For this reason, it is important that the pH of the test chemical group in the growth control and test recepticles be controlled to provide the appropriate initial conditions, especially where the antimicrobial product has a relatively high pH (acidic). For that reason, it is preferred to add a selected pH buffer, such as a mixture of sodium dihydrogen phosphate and sodium hydrogen phosphate, to the set of test chemicals in the wells. The amount of this buffer is preferably kept low to avoid exacerbating the autoreduction tendencies of the growth medium. In addition, it has been discovered that, during incubation, the growth medium itself tends to reduce resazurin and for that reason a redox stabilizer such as potassium ferrocyanide is included in the set of test chemicals disposed in the three wells. The redox stabilizer utilized must not be toxic to the microorganism in order not to interfere in the growth process.

### The Visible Light Reading Protocol (Figs. 4-8)

The visible light reading protocol used in accordance with this invention is based on the color shift from blue to red that is produced during the reduction of resazurin in the test well to resorufin as a result of microorganism growth. If there is growth of the microorganism in the test well 103 despite the presence of the preselected concentration of antimicrobial product disposed therein, then the test chemical solution present in the test well will turn from blue to red and a simple visual inspection of the test well provides a basis for determining a positive or negative test result. The growth control wells provide a basis for comparison of growth and no growth conditions to assist in identifying the condition of the test well.

Figs. 4-8 illustrate in more detail the visible light reading protocol. Fig. 4 illustrates the initial condition of the test pancl 100A prior to incubation. All three of the wells are blue in color as indicated by the shading in the area representing the wells.

The visible light reading protocol includes panel reading qualification algorithms which are used to determine whether the panel itself has failed to provide a proper basis for an accurate test or something has gone wrong and precludes achieving an accurate determination of organism growth or no growth in the test well. Fig. 5 illustrates a failed test due to a change in color of the negative growth control well from blue to red. Since no microorganism had been dispensed into that well, it should not have anything growing therein to produce the blue to red color shift from reduction of resazurin to resorufin. The negative growth control well may change slightly in the depth of blue color therein due to some autoreduction of resazurin by the growth medium during incubation, but change to a pink or red color indicates that the test has likely failed must be repeated. The color of the positive growth control well and the test well are not indicated in Fig. 5 since the color of these wells is not involved in this aspect of the panel qualification algorithm.

Fig. 6 illustrates a failed test due to the failure of the positive growth control well to show a color change from blue to red after incubation. The failure of the microorganism to grow in the positive growth control well where there is supposed to be no growth inhibiting test chemicals present means that there is no reliable basis for judging whether growth of the microorganism in the test well has been inhibited or not by the concentration of antimicrobial product present there.

Figs. 7 and 8 illustrate two test panels which have passed the panel qualification tests and also illustrate the algorithm for determining the final test result. In Fig. 7, inspection of the negative growth control well shows that it has remained blue in color as it should since no microorganism is supposed to be present in that well. The positive growth control well has shifted in color from blue to red as it should since the microorganism dispensed thereinto should be growing without any inhibition. In the test well 103, the color of the test solution is also still blue, indicating no organism growth in the test well. Accordingly the test result is negative, i.e. no organism growth in the test well or test recepticle.

In Fig. 8, the negative growth control well and positive growth control well have the proper colors and the red color indicated in the test well produces a positive test result, i.e. there is organism growth in the test well that produced the color shift there just as in the positive growth control well.

### Manual Reading

It will be appreciated that the test panel 100 can easily be read manually, that is by looking at the wells with the naked eye to determine the test results assuming that the person doing the reading has the normal visual acuity for color recognition.

In most cases of visual reading of the test panel, the panel will have been incubated for a sufficient period of time that the reduction of resazurin in the test well to resorufin will have proceeded to the point that the color shift from blue to red will be dramatic and easily discernable. However, in some cases of a weakly growing organism, or under conditions where the concentration of the antimicrobial product in the test well is just on the borderline of the MIC, the organism growth may be slowed to the point that the degree of color shift from blue to red is not strong. A color guide may be provided to aid in interpreting the test results and will illustrate the degree of color shift that must be present to call the test result positive or negative.

The advantage of this invention over the visual reading of susceptibility test panels which rely on detection of turbidity is that the color change from blue to red has been determined to be much easier to detect than small amounts of turbidity. It has been shown in side by side comparison, that amounts of microorganism growth which produce difficult to detect turbidity after overnight incubation of the panel usually produce a degree of color shift from blue to red that is easy to detect as an indicator of organism growth. As a result, it takes less skill and concentration to manually read a test panel which utilizes the method of this invention. This results in a faster and more accurate reading of the test results and gives the technician more confidence in reporting the test results.

### Automated Reading

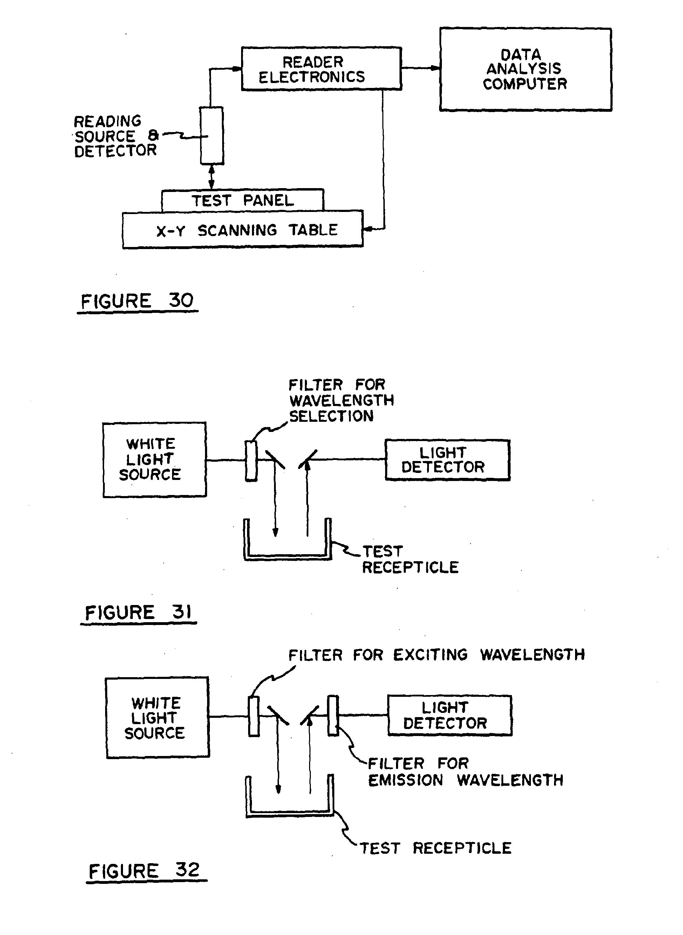

Automated reading of a test panel using the visible light reading protocol can also be readily achieved by instrumentation that is capable of colorimetric determinations. A schematic diagram of an automated colorimetric reading system is shown in Figs. 30 and 31 for the case where the test panel is an opaque material and the white light source is above the pancl. An alternative system in which the panel is clear and the white light source and filter are below the panel could also be used.

As illustrated, in each case the test panel 100 is scanned relative to the light source and detector so that each of the wells is read in sequence. After data on the color of the test solution in each of the three wells is obtained, the algorithms of the visible light reading protocol are applied to the data. First, the panel qualification algorithms are applied in the same manner as in the visual reading approach above and then the test well data is examined to determine the final test result if the qualification tests are passed. With automated reading, it may be possible to accurately quantify the respective degrees of color difference between the test well and the negative growth control well and between the test well and the positive growth control well and to use that quantified data as the basis for determining the test result. The algorithm in this case may be very similar to the fluorescence excitation reading protocol which is discussed below.

### The Fluorescence Excitation Reading Protocol

### Single Reading-- Static Testing Protocol

Since resazurin is non-fluorescing whereas resorufin is strongly fluorescing at a wavelength of 580 nanometers, the test panel 100 may be read using a fluorometer in accordance with a fluorescence excitation reading protocol using a reading system illustrated schematically in Fig. 31. An exciting source having a wavelength at 560 or below is used to excite fluorescence emission from resorufin in the wells. Adequate separation of exciting and emission wavelenghts should be maintained. The panel 100 is scanned with respect to the exciting light source and detector so that the value of fluorescence excitation of resorufin in each of the three wells is obtained. For purposes of this explanation, the value obtained from the negative growth control well is designated N, the value from the positive growth control well is designated P, and the value from the test well is designated T. This reading of the panel is done after incubation of the test panel has been done for a period of time sufficient to cause a substantial production of resorufin in wells in which organism growth is occuring. The values of N and P are then examined to determine if the data corresponds with a valid test. The value of N is compared with and for valid panel data must be below a threshold value Nf which has been determined to be indicative of a failed test due to the presence of too much resorufin in the negative growth control well due to contamination of the panel or some other cause. The value of P is compared with, and for valid panel data must be above a threshold value Pf which has been determined to be indicative of a failed test due to the presence of too little resorufin in the positive growth control well after the incubation period due to a failure of the growth medium to promote organism growth or other causes. If the panel data passes these validation tests, then the test well data can be operated on in accordance with the rest of the fluorescence excitation reading protocol.

A growth control parameter Gc is preferably calculated as the difference in the values of P and N. Similarly a corrected test parameter Tc is preferably calculated as the difference in the values T and N. The value N obtained from the negative growth control well is thus treated as a background value of resorufin that may be present in the other two wells due to some autoreduction of resazurin during the incubation process.

The next step in this reading protocol is to calculate the value of a test variable I using a prearranged functional combination of the values of Gc and Tc. This functional combination could be simply the difference between the two values, but it is preferrable to use a function that includes the ratio of Tc and Gc. After the value of the test variable I has been calculated, it is subjected to a decision algorithm and a test result is reported based on the outcome of application of the decision algorithm.

The decision algorithm is based on data obtained from controlled tests on test panels using organisms that produce known test results. For example, the decision algorithm may involve a predetermined positive decision value XP and a predetermined negative decision value XN. These decision values are based on data collected from organisms that behave in a known manner with the value XP selected such that all such known organisms produce a value of the test variable I which is greater than or equal to XP if the organism is growing in the test well or which is less than or equal to XN if the organism is not growing in the test well. The spread between the positive and negative decision values is an indeterminate range and an indeterminate test result will be reported if the value of I is between those two decision values.

### Single reading -- dynamic test protocol

The above described fluorescence excitation reading protocol is basically a static single reading protocol that is done after a predetermined incubation time period associated with the protocol. A more complex, and potentially more accurate fluorescence excitation reading protocol would involve the use of a preliminary panel reading qualification test that required a minimum value difference between P and N, i.e. a minimum value of Gc before the test parameter I is calculated. If the panel passed the other data qualification tests on the P and N values as discussed above, but the value of Gc is below the preset limit, then the panel would be incubated for an additional time period to allow the value of Gc to increase (if it can) with the passage of time. Such a modified approach will give test results of greater accuracy and greater liklihood of avoiding an indeterminate test result for organisms that are slow growing in the positive growth control well even though no antimicrobial product is present.

### Rapid fluorescence excitation reading protocol

The method of this invention is adaptable to a rapid determination of microorganism growth in the test well using a fluorescence excitation reading protocol that is based on determining the dynamic characteristics of the changes in the resorufin content of the growth control wells and the test well. These dynamic characteristics may include the rate of change of the amount of resorufin in these wells (i.e. the velocity of resorufin production) and the rate of change in this rate of change with time (i.e. the acceleration of resorufin production) in the wells.

In test wells in which the microorganism is growing, the number of such microorganisms will increase exponentially with time, producing a corresponding exponential increase in the amount of resorufin in the test well. In test wells in which the microorganism is not growing, some autoreduction of resazurin to resorufin may be occurring, but this occurs at a linear rate and is thus readily distinguishable from growth related resorufin production.

The incubation step in this method involves incubating the three test wells or recepticles together for a time period sufficient to produce a value of a dynamic characteristic of resorufin production in the positive growth control well that exceeds a predetermined panel qualification value. The value of this dynamic characteristic is determined by reading the value of fluorescence excitation of resorufin in both the positive growth control well and negative growth control well at at least two separated time periods (velocity) with at least three measurements at different time periods required for both velocity and acceleration characteristics to be determined. If the value is below the qualification value, the panel is returned for a predetermined further incubation time period after which the values of fluorescence excitation of resorufin are again obtained and the panel qualification test is rerun. This continues until the panel qualifies for reading or it is determined from the data that the panel is defective and will never qualify for reading.

After panel reading qualification tests are passed, the values of the dynamic characteristics of resorufin production already measured are used to calculate values for dynamic production of resorufin in the positive growth control well and the test well by performing additional measurements after one more time period or by using the data values already obtained. The value for the test well is designated T' and the value for the growth control wells is designated G'. The use of these designations with the "prime" signs is not intended to limit the dynamic characteristics to velocity determinations which might be suggested by a strict mathematical interpretation of the use of the "prime" designation. It should be understood that both rate of change and acceleration in rate of change may be used in the values T' and G'.

After these values are determined, the value of a test variable I is calculated as a prearranged functional combination of T' and G', preferable a function including the ratio of these two parameters. Then a test result is reported using the value of the test variable in a preselected decision algorithm. The decision algorithm may utilize predetermined positive and negative decision values XP and XN as used in the visible light reading protocol. Again these values are determined empirically from data obtained in controlled tests using organisms that produce known test results.

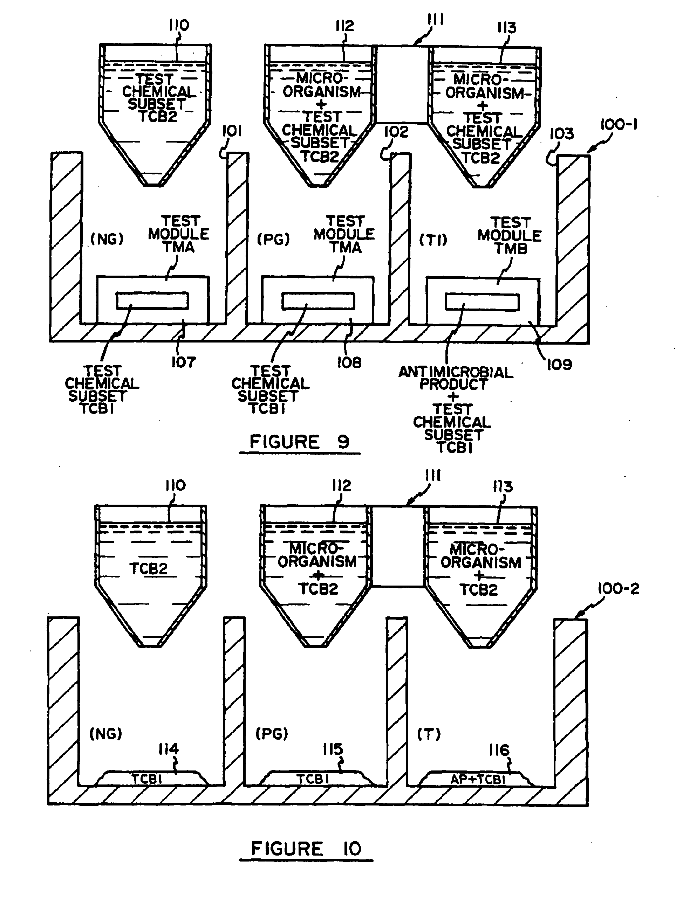

### Test Modules and Test Chemical Subsets (Figs. 9-12)

Fig. 9 illustrates that the general method of this invention is preferably carried out by forming a test module in each of the growth control wells 101 and 102 and the test well 103. As shown, test modules 107 and 108 formed in each of the growth control wells with the test module in each case being a type TMA. A test module 109 is formed in the test well 103 and is of type TMB indicating that it has a different set of test chemicals therein. Each of the test modules 107 and 108 has a test chemical subset TCBI therein and the test module 109 has this same test chemical subset plus the antimicrobial product. The test chemical subsets comprise dry solid volumes of a subset of the constituents of a set of test chemicals which preferably include all of the test chemicals described above, namely resazurin, growth medium, buffer, and a redox stabilizer.

After the test modules are formed in the wells, they are rehydrated by dispensing a volume of liquid into each well. The negative growth control well 101 is rehydrated with a volume of liquid 110 containing only the test chemical subset TCB2. Both the positive growth control well and the test well are rehydrated with volumes of liquid 112 and 113 in inoculator 111 which contain the microorganism as well as the test chemical subset TCB2. The division of the test chemicals between the test modules and the rehydrating liquid illustrates that there is substantial flexibility in the method of this invention in the process of achieving the final test chemical solution in each of the wells prior to the incubation step. The preferred set of test chemicals can be divided into various subsets with one subset in the test module and the other subset in the rehydrating solution. The preferred method involves placing all of the test chemicals in the test modules in the wells so that the rehydrating liquid is simply a volume of sterile liquid for the negative growth control well and a volume of the same liquid with a dispersion of the microorganism therein as an inoculum for the other wells. It should be understood that the test modules could be rehydrated and inoculated in separate steps.

In the preferred method, the test chemical subset in each of the test modules in the growth control wells is the same. However, it should be understood that the method of this invention could be implemented using a different test chemical subset in the negative growth control well from that in the positive growth control well. It would also be possible to use different test chemical subsets in the positive growth control well and the test well, but this would complicate the preparation of the rehydrating inoculum liquid and thus is not the preferred approach.

### Alternate Forms of Test Modules

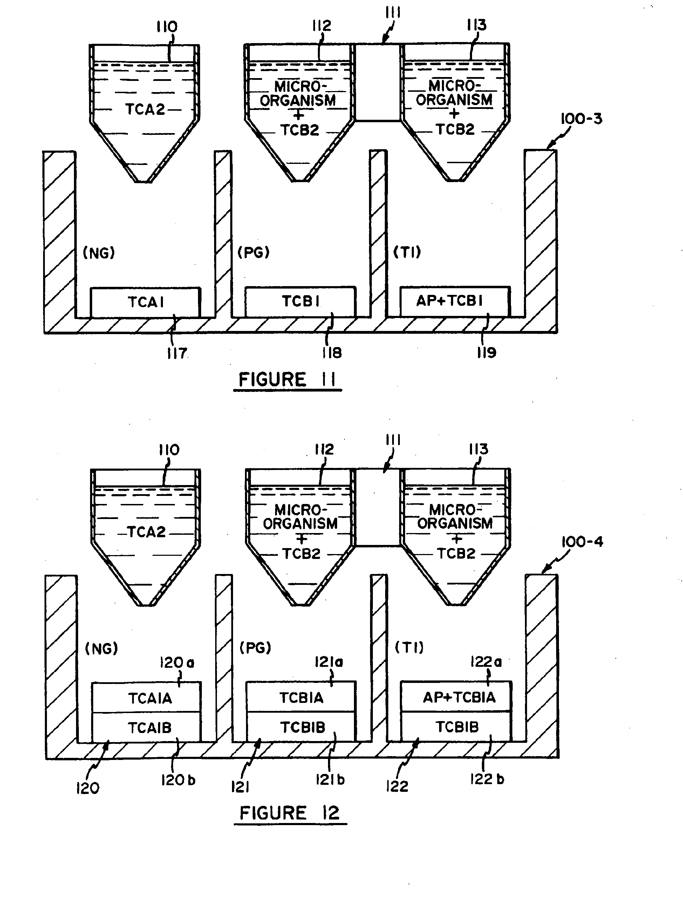

As illustrated in Fig. 10, the test modules in the wells may take the form of a dry solid volume of the test chemicals formed directly on the walls of the wells of the panel 100-2 itself. This can be implemented by dispensing the constituents of the test chemical subset TCBI into each of the growth control wells and that subset plus the antimicrobial product into the test well and then drying the panel, e.g. in a freeze drier, to capture the test chemicals as a dry solid volume on the walls of the wells.

Fig. 11 illustrates that the test modules formed in the wells may take the form of a carrying medium such as the carrying media 117, 118, and 119. The preferred form of the carrying medium in each case is an absorbent paper disk of the same type as is used in the disk diffusion testing described above. Other forms of carrying media may also be used if they have generally comparable characteristics to the absorbent paper disks. The carrying media needs to be capable of being dispensed in a convenient way for manufacture of the test panels.

A process for preparing test modules using absorbent paper disks is described below. In Fig. 11, the a single carrying medium is used in each well, whereas in Fig. 12 a two carrying media are used in each well, with each carrying medium having a portion of the subset of test chemicals formed in the test module carried therein. This illustrates that it is possible, for example, to implement the method of this invention by using two absorbent paper disks, one carrying the resazurin and the other the growth medium. This approach avoids the interactions between these two test chemical constituents in the process of forming the test modules in the wells and especially during the drying of the disks.

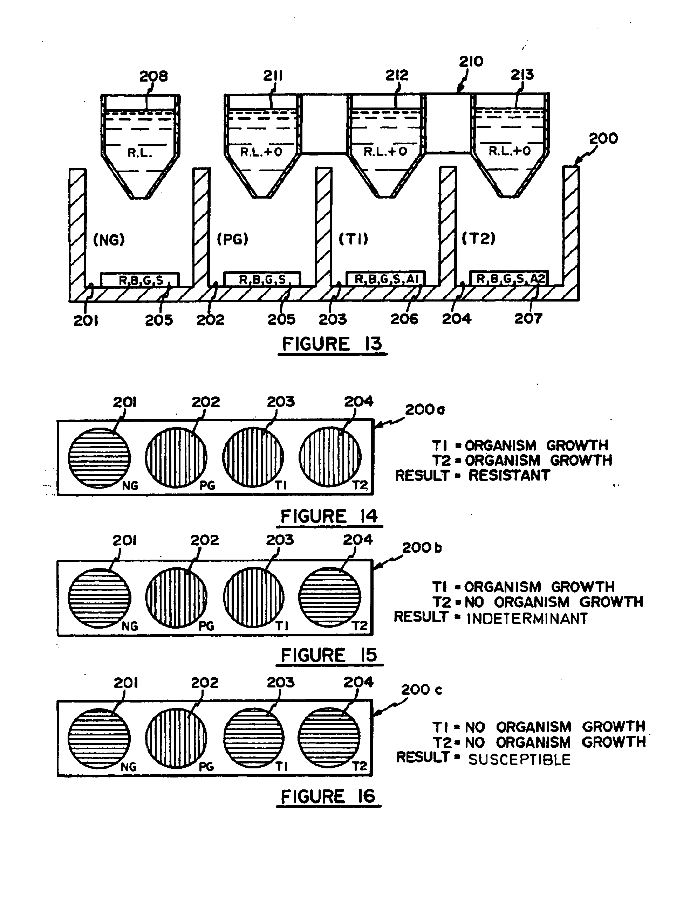

### Qualitative Susceptibility Testing Apparatus (Figs. 13-16)

Figs. 13-16 illustrate the application of the principles of this invention in qualitative susceptibility testing apparatus, i.e. testing for the qualitative susceptibility of a microorganism to growth inhibition by an antimicrobial product utilizing a prearranged qualitative susceptibility testing protocol involving first and second quantities of the antimicrobial product. A test panel 200 defines a negative growth control well 201, a positive growth control well 202 and a pair of test wells 203 and 204. A test module 205 is carried in each of the growth control wells. Test modules 206 and 207 are carried in the pair of test wells.

Fig. 13 illustrates the preferred form of the invention in which the test modules have all of the chemical constituents of the set of test chemicals therein, but it should be understood that the test chemicals may be divided between the test modules and the rehydrating liquid volumes as previously described. Fig. 13 also illustrates the use of a single absorbent paper disk as a carrying medium forming the basis of each test module, but it should be understood that any of the forms of test modules previously discussed could also be used in the qualitative susceptibility testing apparatus in accordance with this invention.

Each of the test module is labelled as containing R for resazurin, B for buffer, G for growth mcdium and S for redox stabilizer. In addition, test module 206 containes a first quantity of the antimicrobial product designated Al and test module 207 contains a second quantity of the antimicrobial product designated A2. For this description, we will consider A2 as the higher of the two concentrations of antimicrobial product in the qualitative susceptibility testing protocol.

Test module 201 in the negative growth control well is rehydrated with a volume of rehydrating liquid portion 208 forming part of an overall inoculating system 210. Each of the test modules 205, 206, and 207 is adapted to be rehydrated with a volume of rehydrating liquid together with a suspension of the microorganism to form the inoculum for those wells. After rehydration, test panel 200 is placed in an incubator for a prearranged incubation time period associated with a preselected reading protocol to be used with the panel.

### Reading the Test Results

Figs. 14-16 illustrate a visible light reading protocol for test panel 200. It should be understood that all of the manual and automated reading protocols described above could be applied to the reading of panel 200.

Referring back to Figs. 4-6, it should be understood that the pre-incubation color in all of the wells of the test panel is blue. Also the same panel reading qualification tests are applied using the negative growth control well and the positive growth control well. These are not repeated here to avoid redundancy and this description assumes that the panel has passed qualification tests for accurate reading with the negative growth control well showing blue color and the positive growth control well showing red color.

In Fig. 14, the panel 200A shows red color in both of the test wells 203 and 204 indicating organism growth in both wells. The corresponding test result of this qualitative susceptibility testing is that the microorganism tested is resistant, i.e. it is not susceptible to growth inhibition by the higher concentration A2 of antimicrobial product in test well 207. In Fig. 15, the panel 200B shows redcolor in test well 203 indicative of organism growth, but blue color in test well 204 indicative of inhibition of growth. In this case the result is indeterminant since the microorganism is neither sensitive or resistant. The use of the term indeterminant should not be misunderstood as failing to give information on the qualitative susceptibility of the microorganism. This test result merely means that the organism is neither highly susceptibility or highly resistant and this gives the referring physician an indication that successful drug therapy may be achieved with that antimicrobial product, in fairly high concentrations.

In Fig. 16, the panel 200C shows blue color in both of the test wells 203 and 204 indicative of inhibition of growth of the microorganism in both test wells. The corresponding test result is "susceptible" indicating that the microorganism can be inhibited in growth by relatively low concentrations of the antimicrobial product.

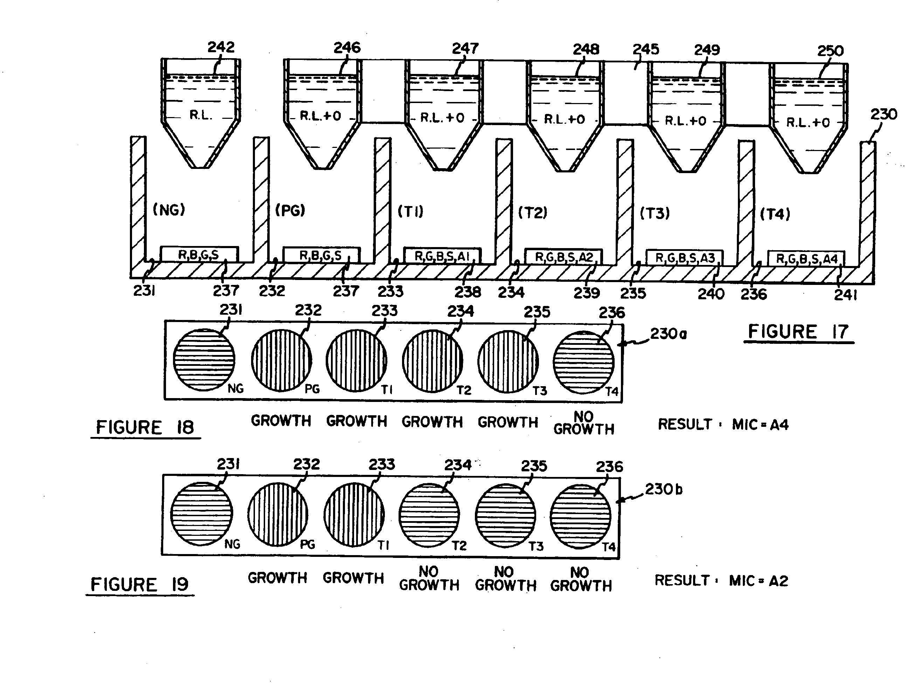

Quantitative Susceptibility Testing Apparatus (Figs. 17-19) Figs. 17-19 illustrate the application of the principles of this invention in quantitative susceptibility testing apparatus, i.e. testing for the quantitative susceptibility of a microorganism to growth inhibition by an antimicrobial product utilizing a prearranged quantitative susceptibility testing protocol involving N different quantities of an antimicrobial product where N is greater than two, and is typically six or more. The current FDA standard for quantitative susceptibility testing is a minimum of five dilutions of the antimicrobial product covering some portion of the therapeutic human dosage range.

Test panel 230 defines a negative growth control well 231, a positive growth control well 232 and, in this case, four test wells 233-236. Four test wells are used here for convenience of illustration. The use of a larger number of test wells will be discussed below in connection with testing kits that incorporate the quantitative susceptibility testing principles of this invention. A test module 237 is carried in each of the growth control wells. Four different test modules 238-241 are carried in the four test wells. As with the above description of qualitative susceptibility testing, the test modules here illustrate the preferred form of the invention using a single carrying medium in the form of an absorbent paper disk with all of the constituents of the set of test chemicals carried in each of the test modules and with the test modules in the test wells also carrying four different quantities of the antimicrobial product, namely A1-A4. For purposes of this description, the amounts of the antimicrobial product will be considered to be increasing from Al to A4. It should be understood that any of the alternative forms of the invention as described above in connection with Figs. 9-12 could also be used here.

Test module 231 is rehydrated with a volume of rehydrating liquid 242 forming a portion of an overall inoculation system 245. Each of the test modules in the other wells is rehydrated with a volume of rehydrating liquid to which a of the microorganism has been added with individual inoculation volumes 246-250 for the wells. A specific form of inoculation system will be described below in connection with a test kit embodiment of this invention. After rehydration and inoculation, panel 230 is placed in an incubator for a prearranged incubation time period associated with a preselected reading protocol to be used with the panel.

### Reading the Test Results as MIC Value

Figs. 18 and 19 illustrate a visible light reading protocol for test panel 230. It should be understood that all of the manual and automated reading protocols described above could be applied to the reading of panel 230.

Referring back to Figs. 4-6, it should be understood that the pre-incubation color in all of the wells of the test panel is blue. Also the same panel reading qualification tests are applied using the negative growth control well and the positive growth control well. These are not repeated here to avoid redundancy and this description assumes that the panel has passed qualification tests for accurate reading with the negative growth control well showing blue color and the positive growth control well showing red color.

As shown in Fig. 18, panel 230A shows a color shift from blue to red in each of the first three test wells 233-235 indicative of growth of the microorganism in each of these test wells. Test well 236 shows the original blue color indicative of no growth of the microorganism in that test well. The test result obtained from this reading is that the MIC of the antimicrobial product used in the test panel is the concentration A4. It should be understood that the concentration A4 refers both to the quantity of the antimicrobial product in the test module and to the concentration of the antimicrobial product in the final test solution after inoculation and rehydration of the test chemicals.

As shown in Fig. 19, panel 230 B shows a color shift from blue to red only in the first test well 233 and the original blue color is present in the other three test wells 234-236. This indicates growth of the microorganism only in the first test well and inhibition of growth in the other three for a resultant MIC value of A2. A2 is the MIC value since the test shows A2 as the lowest concentration of the antimicrobial product which inhibits growth of the microorganism.

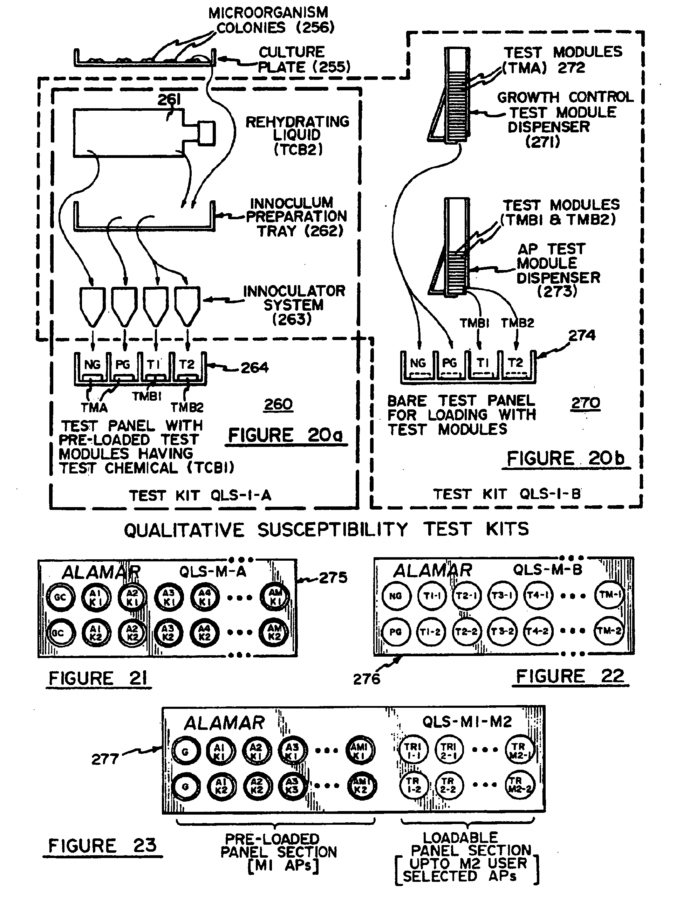

Qualitative Susceptibility Test Kits (Figs. 20-23) Figs. 20-23 illustrate qualitative susceptibility test kits according to this invention and also show how components of the test kits are used in performing the method of this invention. For completeness, Fig. 20 shows a primary culture plate 255 on which colonies of the microorganism to be tested are grown, but this primary culture plate is not considered a part of the test kit of this invention. The components of test kit 260 shown in Fig. 20A are a container 261 of rehydrating liquid, an inoculum preparation tray 262, an inoculator system 263 and a test panel 264 in which the test wells are preloaded with appropriate test modules as previously described.

The rehydrating liquid in container 261 contains the subset of test chemicals TCB2 that are not in the test modules in the wells of the panel 264 as previously described. In the preferred embodiment, all of the test chemicals in the set are in the test modules and the rehydrating liquid is a preselected sterile liquid such as distilled water. It may also comprise 0.9 percent sodium chloride solution and may include a variety of wetting agents to assist in producing a uniform suspension of the microorganisms.

Inoculum preparation tray 262 may be formed in a convenient shape for mixing the rehydrating liquid with microorganisms from colonies growing on the primary culture plate to form the inoculum for the positive growth control well and the test wells T1 and T2. The configuration of the inoculum preparation tray needs to be adapted to the structure and operation of the inoculator system 263. Inoculator system 263 may comprise a standard multi-tip pipetter system or it may be especially designed for dispensing inoculum into the wells of the test panel. As shown, the inoculator system includes an inoculator means for dispensing rehydrating liquid into the negative growth control well and means for dispensing inoculum into the positive growth control well.

Test panel 264 carries preloaded test modules as shown, with two test wells for qualitative susceptibility testing using a single antimicrobial product in this case. The test kit is designated QLS-I-A indicating qualitative susceptibility testing with one antimicrobial product. Fig. 21 illustrates a preloaded test panel 275 useful for qualitative susceptibility of a microorganism using M different antimicrobial products with a pair of test wells associated with each antimicrobial product and preloaded with test modules having appropriate concentrations of the antimicrobial product. As shown in Fig. 21, each of the test modules is preferably a single absorbent paper disk which is labelled with a visually readable legend designating the name of the antimicrobial product in the test module and the concentration of that antimicrobial product therein. the designation Al represents the name of the first antimicrobial product printed on the disk and the designation K1 represents the concentration printed on the disk.

Fig. 20B illustrates a test kit 270 designated QLS-I-B, in which a bare test panel 274 is used instead of a preloaded test panel as in kit 260. Additional components of kit 270 comprise a growth control test module dispenser 271 for dispensing test modules TMA 272 into the negative growth control well and the positive growth control well of panel 274 and an AP test module dispenser 273 for dispensing test modules TMBI and TMB2 into the test wells T1 and T2. Dispenser 273 stores the two different test modules in alternate interleaved fashion so that two sequential actuations of the disk dispensing mechanism are involved in loading the two test wells. The dispensers 271 and 273 may be standard antibiotic disk dispensers loaded with test module in accordance with this invention in the form of absorbent paper disks.

As an alternative to interleaved disk, single dispenser shown in Fig. 20B, it should be apparent that two separate dispensers could be employed, each storing and dispensing one of the AP test modules into an associated test well. It should also be understood that kit 270 may comprise a multiplicity of test panels 274 to go with the dispensers which are capable of dispensing test modules into a number of test panels.

Test panel 274 is configured for testing with one antimicrobial product. As shown in Fig. 22, a test panel 276 that defines a negative growth control well, a positive growth control well and a plurality of pairs of test wells for a plurality of antimicrobial products could also be employed in the test kits of this invention. To load test panel 276, one dispenser of the type 271 is employed for the growth control wells and one one dispenser of the type 273 for each of the antimicrobial products is employed to load the test wells for each antimicrobial product. For test panel 276 it would be preferable to provide a test module dispenser that is capable of loading an entire row of wells at one time.

Fig. 23 illustrates that the test kits of this invention for qualitative susceptibility testing of a microorganism against multiple antimicrobial products may utilize a panel 277 which includes both a preloaded panel section and a loadable panel section. A test kit with this type of test panel combines the convenience of preloaded test wells for antimicrobial products that are conventionally used in virtually all testing situations with the flexibility of user selected antimicrobial products for purposes of customizing a portion of the test panel with antimicrobial products that are tailored to the needs of the user in connection with particular testing situations. Test kits with loadable test wells are especially advantageous in providing for configuring test panels to include newer antimicrobial products as they are developed without waiting for such antimicrobial products to be included on preloaded panels from the manufacturer.

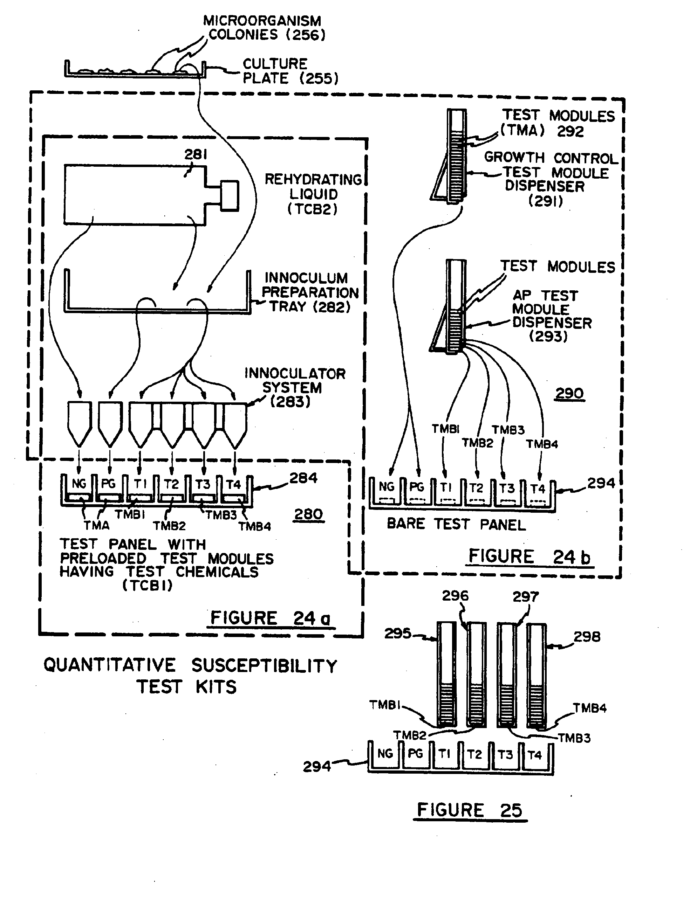

### Quantitative Susceptibility Test Kits (Figs. 24-29)

Figs. 24-29 illustrate quantitative susceptibility test kits according to this invention. Referring to Fig. 24A, test kit 280 illustrated therein comprises a container 281 of rehydrating liquid, an inoculum preparation tray 282, an inoculator system 283 and a preloaded test panel 284. The form and function of these test kit components is generally the same as corresponding components already described in connection with the test kit in Fig. 20A and the description need not be repeated here.

Fig. 24B illustrates a test kit 290 similar to the kit 280 in Fig. 24A but employing a bare test panel 294 and including test module dispensers 291 and 293. Dispenser 291 dispenses growth control test modules into the growth control wells of panel 294. Dispenser 293 dispenses AP test modules into the test wells of panel 294. As shown, dispenser 293 has the test modules for the four test wells stacked in order so that four sequential actuations of the dispenser are used to dispense four different test modules in the form of disks into the four test wells. Fig. 25 illustrates the alternative of using four separate dispensers 295-298 each containing a single type of AP test module with a single concentration of the antimicrobial product therein. These four dispensers can be operated individually or they may be combined in an overall dispensing system which holds all four dispensers in position for dispensing four disks simultaneously and has a single actuator mechanism that operates the dispensing finger in each dispenser at the same time.

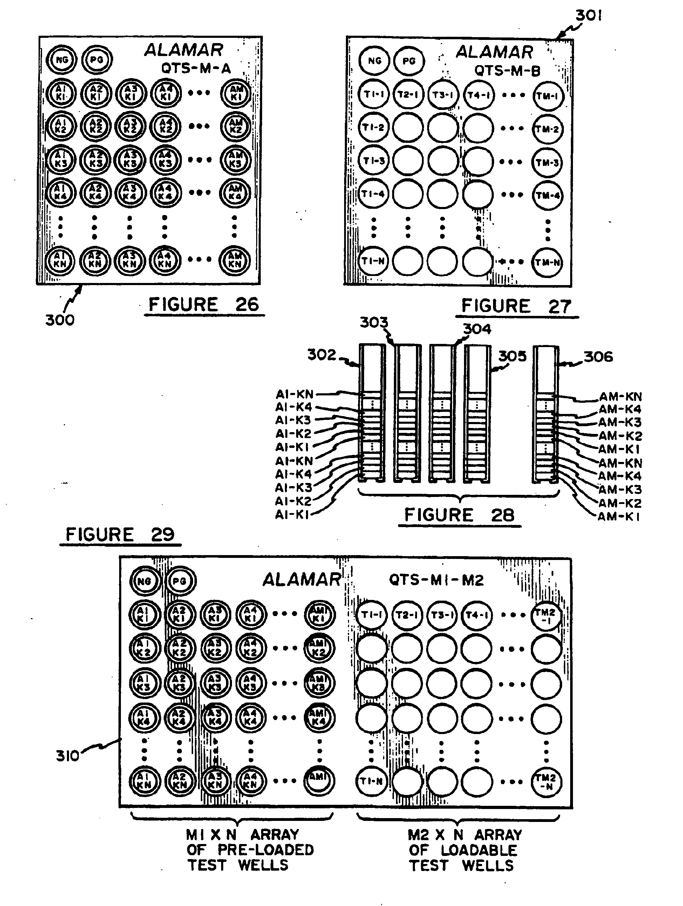

Fig. 26 illustrates a preloaded test panel 300 defining a negative growth control well, a positive growth control well and a M column by N row array of test wells with each column associated with one of a plurality of antimicrobial products and being preloaded with test modules having appropriate antimicrobial products and different concentrations as indicated.

Fig. 27 illustrates a corresponding loadable test panel 301 having the same overall testing capability. As shown in Fig. 28, M individual dispensers 302-306, each having a sequential stacking of AP test modules associated with user selectable antimicrobial products may be employed to load test panel 301.

Fig. 29 illustrates a test panel 310 having an Ml by N array of preloaded test wells and an M2 by N array of loadable test wells. The test module dispensers shown in Fig. 28 may be used to dispense AP test modules into the loadable test well section of the'panel 310. Test panel 310 provides the same advantages in the quantitative susceptibility testing area as the test panel 277 for the qualitative susceptibility testing area as described above.

### Component Features and Manufacturing Processes

### Test Panels

The multiwell test panels used in connection with this invention are preferably formed from a white, opaque plastic material. Such panels allow use of lower concentrations of resazurin for both visible light reading and fluorescence reading. Lower concentrations of resazurin are less toxic to microorganisms and thus minimize potential influence of resazurin on test results. Light intensity reflected from the white walls of the panel increases the signal available in both visible light and fluorescence reading. The white panel provides a uniform reading background which eliminates any need for background lighting equipment for reading the panel and results in more consistent and accurate visual interpretations. Smaller differences in color can be discerned with white background.

White panels can be manufactured with less expensive plastics and plastic panel forming technology because optical clarity is not a requirement.

### Inoculator and Rehydrations Systems

If this invention is used in frozen panels, the test wells may simply be inoculated with a multiprong inoculum transfer devices which transfer a small volume (5-10 ml) from an inoculum seed trough to each test well in the panel.

If this invention is used in dried panels, there are two optional approaches to inoculation. All of the wells of the panel can first be rehydrated with a volume of rehydrating liquid that has no microorganism in it. Then inoculation of the test wells is performed as described above in connection with the frozen panel.

Alternatively, rehydration and inoculation can be done simultaneously by first putting a prearranged concentration of the microorganism in the rehydrating liquid and then dispensing a consistent volume of this inoculum liquid into each test well. This can be done with a single tip pipettor, a multitip pipettor, or with a special delivery system custom designed for this purpose.

### Test Modules

This invention involves placing all of the components of the set of test chemicals, i.e. resazurin, growth medium, buffer (if needed), and redox stabilizer into a test module in the test wells of the panel. To simplify the overall description here, the discussion of processes of making the test modules and panels incorporating test modules will be limited to this approach.

### Frozen Panels

To incorporate the method and apparatus of this invention in frozen test panels, the resazurin together with appropriate stabilizing components of the test chemical group are dispensed into the test wells either together with the growth medium and antimicrobial product dilutions or separately.

### Dried Panels

The process of forming test modules in dried panels is the same as for frozen panels except the test chemical constituents are dried in the wells to form a test module in the form of a dry solid volume on the walls of the wells themselves. Drying can be done with forced air or in vacuum. The formulation of the test chemical components is more critical when the test module is in dried form since the concentrations of the resazurin, antimicrobial product and growth medium become very high as drying nears completion. Adequate buffering of the pH of the solution and stabilization of the reducing action of the growth medium is important under these circumstances. The chemical formulations described below for use in the process of manufacturing test modules in the form of absorbent paper disks are preferably used in dried panels to reduce the volume of liquid in the test wells that must be dried.

### Absorbent Paper Disks

There are various approaches that can be taken to manufacturing paper disks with the components of the test chemical set captured in dried form in the disk. Generally, for large volume manufacturing, sheets of the paper disk media preprinted with the legend identifying antimicrobial product and concentration will be batch impregnated with the test chemical solution, dried, and then cut into disks and packaged. In the case of stacked, serial dilution disk packaging, stacks of paper media with the different concentrations of the antimicrobial product are preferable cut into disks in one operation and then packaged.

To manufacture disks in small volumes, the following process can be employed. A volume of the disk loading solution is made up. Since the paper disks are capable of holding only 25 microliters of the solution and the final volume of the rehydrating liquid in the test wells is conveniently 100 microliters, a four times concentration of the test chemicals is used in the disk loading solution.

Specifically, the following disk loading solution is prepared. The basic carrying solution is phosphate buffer of pH 7.4 in 0.1 molar concentration. The growth medium, which is standard Mueller-Hinton broth in dry powder form, is added at 88.0 grams per liter. Resazurin is added to achieve a concentration of 0.02 grams per liter. Potassium ferrocyanide to serve as a redox stabilizer is added to a concentration of 0.004 molar. This solution is then used as the diluent for the antimicrobial product to be included in the test module disk. The initial concentration of the antimicrobial product is also four times the final concentration desired in the test well after rehydration, but is adjusted to correct for irreversible binding of the antimicrobial product to the disk. In other words, when the dried test chemicals in the disk are rehydrated, the entire amount of antimicrobial product does not enter the rehydrating liquid. The bound antimicrobial product in the disk is not active against the microorganism.

The concentration of resazurin is selected to produce a bright blue starting color for high visual contrast between positive and negative growth of the microorganism. Higher concentration of resazurin would having increasing toxicity for some microorganisms and would decrease or delay the discernable visual color change in response to growth of the microorganism. Lower concentration of resazurin would result in poor contrast between positive and negative test well reactions, i.e. the color in wells where the microorganism is growing to a slight extent would not be as readily discernable as a color change. These statements all pertain to visual reading of the test panels and the concentration of resazurin is less critical for fluorescence excitation reading protocols.

The concentration of growth medium is the standard concentration used in these types of test panels and there is no reason to change this. A decrease in concentration will decrease growth rates and an increase does not increase growth rate, but exacerbates the autoreduction tendency of the growth medium during incubation and drying. It should be understood that other growth medium formulations than the standard Mueller-Hinton could also be used, but they must meet the performance characteristics of the now standard Mueller-Hinton broth.

The buffer is used in sufficient concentrations to prevent pH shifts and accompanying color non-uniformity between test wells or test modules due to high concentrations of acidic antimicrobial products especially during the drying process. The pH 7.4 is used because it is the recommended pH for Mueller-Hinton broth when used in this application. Concentration of buffer is kept at the minimum required to provide stability of pH especially during drying. Too high a molar concentration is to be avoided because it can delay the reduction of resazurin to resorufin and adversely affect the consistency of test results.

The redox stabilizer is used in sufficient concentration to suppress autoreduction of the resazurin during the drying and incubation processes to acceptable levels. Potassium ferrocyanide was selected for its relative low toxicity to microorganisms and its stabilizing capacity in the appropriate oxidation/reduction potential range. Concentration is selected to avoid excessive stabilization of the oxidation/reduction reaction due to microorganism growth since that would delay and/or adversely affect accuracy of test results.

The process of loading the disk loading solution in the disks involves aseptic processing using sterile raw materials. A group of disks is placed in a single layer, without edges touching, in a flat, sterile, container which can be covered, such as a covered Petrie dish. A micropipetter is used to dispense 25 microliters of the disk loading solution onto each disk.

The container is then covered and placed in a freezer at -70 degree Centigrade overnight. The container is then transferred to a freeze drying chamber and the disks are dried in vacuum. They are then removed, placed in capped vials containing a dessicant capsule and stored in a dark refrigerated environment.

For loading disks into test recepticles, the disks are transferred individually in an aseptic manner. It should be understood that this method can be used for small volume production of test panels for use in clinical testing and the like, but large scale automated production of disks and automated panel loading technology would be employed for manufacturing preloaded test panels in volume.

### Automated Reading of Test Panels (Figs. 30-34)

Fig. 30 illustrates the general components employed in an automated reader system for reading test panels which incorporate the methods and apparatus of this invention using either the visible light reading protocol or the fluorescence excitation reading protocol. After incubation the test panel is placed on a scanning table which places each test well in place to be read by the reading source and detector together with the reader electronics. The data from the reader electronics is preferably communicated to a data analysis computer where the algorithms of the associated reading protocol are applied to the data from each well in the test panel.

Fig. 31 shows that, in the case of visible light reflectance reading for implementing a visible light reading protocol, a single filter may be used for selection of the reading wavelength which will be used to determine the reflected color characteristics of the liquid in the test well. Multiple wavelength analysis could also be used if desired. Fig. 32 illustrates that, in the case of fluorescence excitation reading for implementing a fluorescence excitation reading protocol, separate filters are employed for selecting the exciting wavelength and the emission wavelength. Both visible light reading of reflected color and fluorescence reading can be implemented with instrumentation that is currently commercially available and known to persons familiar with this technology.

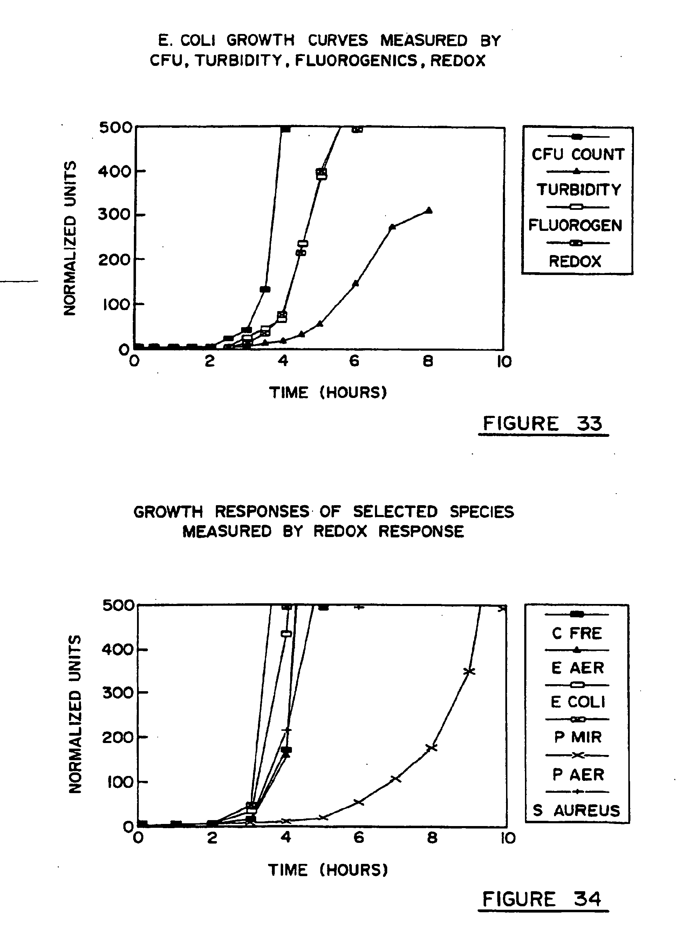

Fig. 33 illustrates that the fluorescence excitation reading of microorganism growth using the resazurin redox reaction in accordance with this invention provides data generally comparable to fluorogenic reading used in prior art rapid microorganism growth detection systems in cases where the particular microorganism is well adapted to detection by the fluorogenic detection system. The fluorescence excitation reading approach of this invention will be superior to the fluorogenic reading of the prior art for microorganisms that are not readily detected by such prior art methods.

Fig. 34 shows the data obtained from fluorescence emission reading of growth of a variety of microorganisms and illustrates the general applicability of the method of this invention to rapid growth determination using a fluorescence excitation reading protocol. The graphs show detection of significant quantities of resorufin due to microorganism growth within a four to six hour incubation time period. The design of a particular fluorescence excitation reading protocol and the test and decision algorithms associated therewith involves the collection of data on a number of microorganisms that produce known response and then building the test and decision algorithms such that growth or no growth of unknown organisms can be detected with a high level of confidence. Persons of skill in this art are familiar with the various methods for designing and building such reading protocols and the implementation thereof in connection with this invention involves a straightforward application of known data gathering and protocol generation principles.

**TABLE I.**

| EXAMPLE OF RESULTS OF DISK DIFFUSION TEST | | | |
|---|---|---|---|
| DISK LABEL | FULL NAME OF ANTIMICROBIAL | MEASURED DIAMETER | TEST RESULT |
| E | Erythromycin | 0 mm | Resistant |
| 15 | 15 micrograms | | |
| | | | |
| CL | Cephalothin | 29 mm | Sensitive |
| 30 | 30 micrograms | | |
| | | | |
| VA | Vancomycin | 18 mm | Sensitive |
| 30 | 30 micrograms | | |
| | | | |
| CZ | Cefazolin | 24 mm | Sensitive |
| 30 | 30 micrograms | | |
| | | | |
| P | Penicillin | 10 mm | Resistant |
| 10 | 10 units | | |

**TABLE II.**

| EXAMPLE OF ANTIMICROBIAL PRODUCTS AND CONCENTRATIONS | | | |
|---|---|---|---|
| WELL LABEL | FULL NAME OF ANTIMICROBIAL | CONCENTRATIONS IN WELLS (mcg/ml) | |
| Two Dilutions Section | | Low | High |
| Am | Ampicillin | 0.5 | 8 |
| Cfm | Cefamandole | 8 | 16 |
| Cfz | Cefazolin | 8 | 16 |
| Cz | Ceftizoxime | 8 | 32 |
| Cf | Cephalothin | 8 | 16 |
| C | Chloramphenicol | 8 | 16 |
| Cp | Ciprofloxacin | 1 | 2 |
| Cd | Clindamycin | 0.5 | 4 |
| E | Erythromycin | 0.5 | 4 |
| Gm | Gentamicin | 4 | 8 |
| P | Penicillin | 0.5 | 8 |
| Rif | Rifampin | 2 | 4 |
| Te | Tetracycline | 4 | 8 |
| T/S | Trimethoprim/Sulfamethoxazole | 2 | 38 |
| T/S | Trimethoprim/Sulfamethoxazole | 38 | 152 |
| | | | |

| Single Dilution Section | | Dilution | |
|---|---|---|---|
| GmS | Gentamicin Synergy Screen | 500 | |
| Ox | Oxacillin | 2 | |
| Cp-U | Ciprofloxacin-Urinary | 8 | |
| Fd | Nitrofurantoin | 64 | |
| Nxn | Norfloxacin | 16 | |
| Sx | Sulfamethoxazole | 256 | |

## Claims

1. A method for testing the susceptibility of a microorganism to growth inhibition by a preselected concentration of an antimicrobial product comprising the steps of:
(a) disposing in each of a negative growth control recepticle and a positive growth control recepticle a prearranged concentration of a growth medium for microorganisms and a prearranged concentration of resazurin predetermined to be in a concentration range **characterised by** low toxicity to microorganisms and substantial sensitivity to reduction to resorufin by metabolic products of microorganism growth, and a pre-selected redox stabiliser **characterised by** substantial lowering of the reduction of resazurin by said growth medium during step (g);
(b) disposing in said positive growth control recepticle a prearranged concentration of said microorganism;
(c) disposing in a test recepticle said preselected concentration of said antimicrobial product;
(d) disposing in said test recepticle said prearranged concentration of resazurin, and a preselected redox stabiliser **characterised by** substantial lowering of the reduction of resazurin by said growth medium during step g;
(e) disposing in said test recepticle said prearranged concentration of growth medium;
(f) disposing in said test recepticle said prearranged concentration of said microorganism;
(g) incubating all of said recepticles together for an incubating time period associated with a preselected reading protocol comprising one of a visible light reading protocol and a fluorescence excitation reading protocol; and
(h) after said incubating time period, reading said recepticles in accordance with said preselected reading protocol to determine the presence or absence of growth of said microorganism in said test receptible on the basis of the relative concentrations of resazurin and resorufin therein, said visible light reading including a decision algorithm based on at least one predetermined functional combination of the visible light reflectantance color detected in each of said recepticles, said fluorescence excitation reading protocol including a decision algorithm based on at least one predetermined functional combination of the values of the fluorescence emission signal produced by the reduction product resorufin in each of said recepticles.

2. The method of claim 1, wherein said steps c. through f. are carried out by:
j1) forming in said test recepticle a test module comprising a dry solid volume of prearranged quantity of said antimicrobial product and a dry solid volume of a preselected subset of the constituents of a set of test chemicals comprising said resazurin and said growth medium; and
j2) dispensing into said test well a volume of liquid having therein said prearranged concentration of said microorganism and all of the constituents of said set of test chemicals not in said test module to rehydrate said test module prior to carrying out said step g.

3. The method of claim 2, wherein said set of test chemicals includes a preselected buffer for controlling the final pH of the rehydrated test chemical solution and a preselected redox stabilizer **characterised by** substantial lowering of the reduction of resazurin by said growth medium during step (g).

4. The method of claim 3, wherein step j1) is carried out by
k1) dispensing onto a carrying medium a prearranged volume of liquid having therein said preselected concentration of said antimicrobial product, said prearranged concentration of resazurin, said preselected buffer, and said preselected redox stabiliser;
k2) drying said carrying medium to capture said antimicrobial product, said resazurin, said buffer, and said redox stabiliser therein as dry solid components; and
k3) placing said carrying medium into said test recepticle either before or after said steps k1) and k2) are performed.
and said step j2) is carried out by
I1) dispensing into said test recepticle a volume of liquid having therein said prearranged concentration of said growth medium and said prearranged concentration of said microorganism to rehydrate said dry solid components in said carrying medium.

5. The method of claim 3, wherein said step j1) is carried out by
k1) dispensing onto a carrying medium a prearranged volume of liquid having therein said preselected concentration of said antimicrobial product, said prearranged concentration of said resazurin, said prearranged concentration of said growth medium, said preselected buffer and said preselected redox stabiliser ;
k2) drying said carrying medium to capture said antimicrobial product, said resazurin, said buffer, and said redox stabiliser therein as dry solid components; and
k3) placing said carrying medium into said test recepticle either before or after said steps k1) and k2) are performed.
and said step j2) is carried out by
I1) dispensing into said test recepticle a volume of liquid comprising said prearranged concentration of said microorganism to rehydrate said dry solid components in said carrying medium.

6. The method of claim 1, wherein said step a. is carried out by
j1) dispensing onto each of a pair of carrying media a prearranged volume of liquid having therein said prearranged concentration of said resazurin, said prearranged concentration of said growth medium, a preselected buffer and a preselected redox stabiliser, said preselected buffer having a prearranged concentration for controlling the pH of said liquid;
j2) drying each of said carrying media to capture said resazurin, said growth medium said buffer; and said redox stabiliser therein as dry solid components;
j3) placing one of said carrying media into each of said negative growth control recepticle and said positive growth control recepticle either before or after said steps j1) and j2) are performed; and
j4) dispensing into said negative growth control recepticle a volume of sterile liquid to rehydrate said dry solid components;
and steps c., d., and e. are carried out by
k1) dispensing onto a carrying medium a prearranged volume of liquid having therein said preselected concentration of said antimicrobial product, said prearranged concentration of said resazurin, said prearranged concentration of said growth medium, said preselected buffer and said preselected redox stabiliser;
k2) drying and carrying medium to capture said antimicrobial product, said resazurin, said growth medium, said buffer and said redox stabiliser therein as dry solid components;
k3) placing said carrying medium into said test recepticle either before or after said steps j1) and j2) are performed. and said step b. and f. are carried out by
I1) dispensing into said positive growth control recepticle and said test recepticle a volume of liquid comprising said prearranged concentration of said microorganism to rehydrate said dry solid components in said carrying medium.

7. The medium of claim 1, wherein
said step g. comprises incubating said recepticles together for a predetermined incubating time period associated with a fluorescence excitation reading protocol;
and said step h. comprises reading said recepticles in accordance with said fluorescence excitation reading protocol, including the steps of
h1) reading the value of fluorescence excitation of resorufin in each of said recepticles, said value for said negative growth control well being designated N, said value for said positive growth control well being designated P, and said value for said test recepticle being designated T;
h2) calculating the values of a growth control parameter Gc and a corrected test parameter To as the difference in the values of P and N and the values T and N, respectively;
h3) calculating the value of a test variable I comprising a prearranged functional combination of said growth control parameter Gc and said corrected test parameter Tc, and
h4) reporting a test result using said test variable I in a preselected decision algorithm.

8. The method of claim 7, wherein said step h3) comprises calculating said test variable I as a prearranged function which includes the ratio of Tc and Gc;
and said steps h4) comprises reporting one of
a positive test result (organism growth) if I is greater than or equal to a predetermined positive decision value XP,
a negative test result (no organism growth) if I is less than or equal to a predetermined negative decision value XL, and
an indeterminate test result if I is between XP and XL,
where said decision value XL and XP are determined empirically from data obtained in controlled tests using organisms that produce known test results.

9. The method of claim 1, wherein said step g. is carried out in accordance with a rapid fluorescence excitation reading protocol and includes incubating said recepticles together for a time period sufficient to produce a predetermined rate of growth of said microorganisms in said positive growth control recepticle as determined by reading the value of fluorescence excitation of resorufin in said negative growth control recepticle and said positive growth control recepticle at at least two separated times during said time period;
and said step h. comprises reading said recepticles in accordance with said rapid fluorescence excitation reading protocol which includes the steps of
h1) determining the values of prearranged dynamic characteristics of production of resorufin in each of said test recepticle and said positive growth control recepticle by measuring the values of fluorescence excitation of resorufin therein and in said negative growth control recepticle at at least two separated time periods, using the measured values from said negative growth control recepticle for correction, and designating said values at T' and G', respectively;
h2) calculating the value of a test variable, designated I and comprising a prearranged functional combination of T' and G'; and
h3) reporting a test result using the value of said test variable I in a preselected decision algorithm.

10. The method of claim 9, wherein said step h2) comprises calculating said test variable I as a prearranged function which includes the ratio of T' and G';
and said step h3) comprises reporting one of
a positive test result (organism) if I is greater than or equal to a predetermined positive decision value XP,
a negative test result (no organism growth) if I is less than or equal to predetermined negative decision value XL, and
an indeterminate test result if I is between XP and XL,
where said decision values XL and XP are determined empirically from data obtained in controlled tests using organisms that produce known test results.

11. The method of claim 1, wherein said step g comprises incubating said recepticles together for a predetermined incubating time period associated with a visible light reading protocol; and
said step h. comprises the steps of
h1) qualifying said recepticles for reading based on detecting absence of a discernable shift in the initial blue color attributable to resazurin in said negative growth control recepticle indicating no growth of said microorganism therein and presence of a substantial shift in color of said positive growth control well from initial blue color of said resazurin toward a red color attributable to production of said reduction product resorufin caused by microorganism growth therein;
h2) reporting a failed test if said recepticles do not qualify for reading according to step h1); and
h3) reporting a test result if said recepticles qualify for reading according to step i1) by detecting the presence or absence of a substantial shift in color of said test recepticle from an initial blue color of resazurin toward the red color of resorufin with said test result being positive (organism growth) if said color shift is present and said test result being negative (no organism growth) if said color shift is absent.

12. The method of claim 3, wherein said step j1) is carried out by
k1) dispensing into said test recepticle a prearranged volume of liquid having therein said preselected concentration of said antimicrobial product, said prearranged concentration of said resazurin, said prearranged concentration of said growth medium, said preselected buffer and said preselected redox stabiliser ; and
k2) drying said volume of liquid to capture said antimicrobial product, said resazurin, said growth medium, said buffer and said redox stabiliser as dry solid components;
and said step j2) is carried out by
I1) dispensing into said test recepticle a volume of liquid comprising said prearranged concentration of said microorganism to rehydrate said dry solid components.

13. The method of claim 5, wherein said carrying medium comprises an absorbent paper disk and said dry solid components are captured in said disk.

14. The method of claim 5, wherein said carrying medium comprises at least first and second absorbent paper disks, said first disk having said resazurin therein, said second disk having said growth medium therein, and said buffer and said redox stabiliser being dispensed into one of said first and second disks.

15. The method of claim 5, wherein said step g comprises incubating said recepticles together for a predetermined incubating time period associated with a visible light reading protocol; and
said step h, comprises the steps of
h1) qualifying said recepticles for reading based on detecting absence of a discernible shift in the initial blue color attributable to resazurin in said negative growth control recepticle indicating no growth of said microorganism therein and presence of a substantial shift color of said positive growth control well from initial blue color of said resazurin toward a red color attributable to production of said reduction product resorufin caused by microorganism growth therein;
h2) reporting a failed test if said recepticles do not qualify for reading according to step h1) ; and
h3) reporting a test result if said recepticles qualify for reading according to step I1) by detecting the presence or absence of a substantial shift in color of said test recepticle from an initial blue color of resazurin toward the red color of resorufin with said test result being positive (organism growth) if said color shift is present and said test result being negative (no organism growth) if said color shift is absent.

16. Apparatus for testing the qualitative susceptibility of a microorganism to growth inhibition by an antimicrobial product utilizing a prearranged qualitative susceptibility testing protocol involving first and second quantities of said antimicrobial product, comprising a test panel defining a negative growth control well, a positive growth control well, and a pair of test wells;
a test module carried in each of said negative growth control well, said positive growth control well, and said pair of test wells, each of said test modules comprising a dry solid volume of a subset of the constituents of a set of test chemicals comprising a quantity of resazurin and a quantity of growth medium for microorganisms and a preselected redox stabiliser **characterised by** substantial lowering of the reduction of resazurin by said growth medium, each of said test modules in said pair of test wells further comprising a dry solid volume of one of said first and second quantities of said antimicrobial product;
said test module in said negative growth control well being adapted to be rehydrated by a volume of sterile liquid containing all of the constituents of said set of test chemicals not included in said subset;
said test modules in each of said positive growth control well and said pair of test wells being adapted to be rehydrated by a volume of inoculum liquid containing all of the constituents of said set of test chemicals not included in said subset together with a prearranged concentration of said microorganism;
said volume of sterile liquid and said volume of inoculum liquid having the preselected volume amount, said quantity of resazurin being preselected relative to said preselected volume amount to produce a preselected concentration of resazurin in each of said wells in a range **characterised by** low toxicity to microorganism growth and substantial sensitivity to reduction to resorufin by metabolic products of microorganism growth, and said quantity of growth medium being preselected to produce a preselected concentration of growth medium in each of said wells;
said test panel and said rehydrated test modules carried therein being adapted to be incubated for an incubating time period associated with a preselected reading protocol comprising one of a visible light reading protocol and a fluorescence excitation reading protocol and thereafter to be read in accordance with said preselected reading protocol to determine the presence or absence of growth of said microorganism in said test wells.

17. Apparatus for testing the quantitative susceptibility of a microorganism to growth inhibition by an antimicrobial product utilising a prearranged quantitative susceptibility testing protocol involving N different quantities of said antimicrobial product where N>2, comprising
a test panel defining a negative growth control well, a positive growth control well, and N test wells,
a test module carried in each of said negative growth control well, said positive growth control well, and said N test well, each of said test modules comprising a dry solid volume of a subset of the constituents of a set of test chemicals comprising a quantity of resazurin and a quantity of growth medium for microorganisms and a preselected redox stabiliser **characterised by** substantial lowering of the reduction of resazurin by said growth medium, each of said test modules in said N test wells further comprising a dry solid volume of a different quantity of said antimicrobial product preselected in accordance with said quantitative susceptibility testing protocol;
said test module in said negative growth control well being adapted to be rehydrated by a volume of sterile liquid containing all of the constituents of said set of test chemicals not included in said subset;
said test modules in each of said positive growth control well and said N test wells being adapted to be rehydrated by a volume of inoculum liquid containing all of the constituents of said set of test chemicals not included in said subset together with a prearranged concentration of said microorganism;
said volume of sterile liquid and said volume of inoculum liquid having the same preselected volume amount, said quantity of resazurin being preselected relative to said preselected volume amount to produce a preselected concentration of resazurin in each of said wells in a range **characterised by** low toxicity to microorganism growth and substantial sensitivity to reduction to resorufin by metabolic products of microorganism growth, and said quantity of growth medium being preselected to produce a preselected concentration of growth medium in each of said wells;
said test panel and said rehydrated test modules carried therein being adapted to be incubated for an incubating time period associated with a preselected reading protocol comprising one of a visible light reading protocol and a fluorescence excitation reading protocol and thereafter to be read in accordance with said preselected reading protocol to determine the presence or absence of growth of said microorganism in said test wells.

18. Apparatus as claimed in claim 16 or claim 17, wherein each of said test modules comprises a carrying medium carried in an associated well and each of said test modules carries said subset of the constituents of said set of test chemicals.

19. Apparatus as claimed in claim 18, wherein said carrying medium comprises an absorbent paper disk and each of said absorbent paper disks in said test wells is imprinted with a visually readable designation of both the name and concentration of said antimicrobial product stored therein.

20. Apparatus as claimed in claim 18, wherein said carrying medium comprises at least first and second absorbent paper disks, said first disk carrying said resazurin and said second disk carrying said growth medium, one of said first and second paper disks each of said test wells carrying said associated quality of said antimicrobial product and being imprinted with a visually readable designation of both the name and concentration of said antimicrobial product stored therein.

21. Kit for testing the qualitative susceptibility of a microorganism to growth inhibition by an antimicrobial product utilising a prearranged qualitative susceptibility testing protocol involving first and second quantities of said antimicrobial product, comprising
a test panel defining a negative growth control well, a positive growth control well, and a pair of test wells;
a test module carried in each of said negative growth control well, said positive growth control well, and said pair of test wells, each of said test modules comprising a dry solid volume of a subset of the constituents of a set of test chemicals comprising a quantity of resazurin and a quantity of growth medium for microorganisms and a preselected redox stabiliser **characterised by** substantial lowering of the reduction of resazurin by said growth medium, each of said test modules in said pair of test wells further comprising a dry solid volume of one of said first and second quantities of said antimicrobial product;
a container of dehydrating liquid containing all of the constituents of said set of test chemicals not included in said subset;
inoculum storage means for storing a volume of said rehydrating liquid with said microorganism added thereto to form an inoculum liquid having a prearranged concentration of microorganism therein;
inoculator means including means for dispensing a prearranged quantity of said rehydrating liquid into-said negative growth control well, means for dispensing a prearranged quantity of said inoculum liquid into said, positive growth control well, and said pair of test wells to rehydrate the test chemicals in said test modules therein;
said quantity of resazurin being preselected relative to said prearranged quantity to produce a preselected concentration of resazurin in each of said wells in a range **characterised by** low toxicity to microorganism growth and substantial sensitivity to reduction to resorufin by metabolic products of microorganism growth, and said quantity of growth medium being preselected to produce a preselected concentration of standard growth medium in each of said wells;
said test panel and said rehydrated test modules carried therein being adapted to be incubated for an incubating time period associated with a preselected reading protocol comprising one of a visible light reading protocol and a fluorescence excitation reading protocol and thereafter to be read in accordance with said preselected reading protocol to determine the presence or absence of growth of said microorganism in,said test wells.

22. Kit for testing the quantitative susceptibility of a microorganism to growth inhibition by an antimicrobial product utilising a prearranged quantitative susceptibility testing protocol involving N different quantities of said antimicrobial product where N>2, comprising
a test panel defining a negative growth control well, a positive growth control well, and N test wells;
a test module carried in each of said negative growth control well, said positive growth control well, and said N test wells, each of said test modules comprising a dry solid volume of a subset of the constituents of a set of test chemicals comprising a quantity of resazurin and a quantity of growth medium of microorganisms and a preselected redox stabiliser **characterised by** substantial lowering of the reduction of resazurin by said growth medium, each of said test modules in said N test wells further comprising a dry solid volume of one of said N different quantities of said antimicrobial product;
a container of rehydrating liquid containing all of the constituents of said set of test chemicals not included in said subset;
inoculum storage means for storing a volume of said rehydrating liquid with said microorganism added thereto to form an inoculum liquid having a prearranged concentration of microorganism therein;
inoculator means including means for dispensing a prearranged quantity of said rehydrating liquid into said negative growth control well, means for dispensing a prearranged quantity of said inoculum liquid into said positive growth control well, and each of said N test wells to rehydrate the test chemicals in said test modules therein;
said quantity of resazurin being preselected relative to said prearranged quantity to produce a preselected concentration of resazurin in each of said wells in a range **characterised by** low toxicity to microorganism growth and substantial sensitivity reduction to resorufin by metabolic products of microorganism growth, and said quantity of growth medium being preselected to produce a preselected concentration of standard growth medium in each of said wells;
said test panel and said rehydrated test modules carried therein being adapted to be incubated for an incubating time period associated with a preselected reading protocol comprising one of a visible light reading protocol and a fluorescence excitation reading protocol and thereafter to be read in accordance with said preselected reading protocol to determine the presence or absence of growth of said microorganism in said test wells.

23. A kit as claimed in claim 21 or claim 22, wherein each of said test modules comprises an absorbent paper disk and each of said absorbent paper disks in said test wells is imprinted with a visually readable designation of both the name and concentration of said antimicrobial product stored therein.

24. A kit as claimed in claim 21 or claim 22, wherein each of said test modules comprises at least first and second absorbent paper disks, said first disk carrying said resazurin and said second disk carrying said growth medium, one of said first and second paper disks in each of said test wells carrying said associated quantity of said antimicrobial product and being imprinted with a visually readable designation of both the name and concentration of said antimicrobial product stored therein.

25. A kit comprising a first test module as claimed in claim 21, a second test module comprising a carrying medium for carrying in dry solid form said second quantity of said antimicrobial product and said test chemical group; and
dispensing means for dispensing said first and second test modules into said pair of test wells.

26. A kit as claimed in claim 25 further comprising
a pair of growth test modules each comprising a carrying medium for carrying in dry solid form said test chemical group; and
a container of said rehydrating liquid;
said dispensing means includes means for dispensing one of said pair of growth test modules into each of said negative growth control well and said positive growth control well; and
said inoculator means includes means for dispensing a prearranged quantity of said rehydrating liquid into said negative growth control well and a prearranged quantity of said inoculum into said positive growth control well.

27. A kit as claimed in claim 25 comprising a multiplicity of test panels for testing multiple microorganisms and a multiplicity of each of said first test module and said second test module; and
said dispensing means comprising first and second storage means for storing said multiplicity of first and second test modules, respectively, and a dispenser operatively associated with said storage means for simultaneously dispensing one of said first test modules from said first storage means into one of said test wells and one of said second test modules from said second storage means into the other of said test wells in one test panel of said multiplicity of test panels.

28. A kit as claimed in claim 25 comprising a multiplicity of test panels for testing multiple microorganisms and a multiplicity of each of said first test module and said second test module; said dispensing means comprising storage means for storing said multiplicity of first and second test modules in an interleaved manner, and a dispenser operatively associated with said storage means for alternatingly dispensing a first test module into one of said test wells in one of said test panels and a second test module into the other of said test wells in said one of said test panels.

29. A kit as claimed in claim 23 wherein said set of test chemicals further comprises a preselected buffer for pH control and wherein all of the constituents of said set of test chemicals are carried in said absorbent paper disk, said inoculum liquid containing only said microorganism.

30. A kit as claimed in claim 25 adapted for testing the qualitative susceptibility of said microorganism to growth inhibition by M different antimicrobial products, wherein
said test panel defines M pairs of test wells, each pair being associated with one of said M antimicrobial products;
M of said first test modules and M of said second modules are provided, each being associated with one of said antimicrobial products;
said dispensing means comprises means for dispensing each of said M first test modules and each of said M second test modules into associated ones of said M pairs of test wells; and
said inoculator means dispenses a prearranged quantity of said inoculum liquid into each well of said M pairs of test wells.

31. A kit as claimed in claim 30 wherein each of said M first and second test modules comprises an absorbent paper disk imprinted with a visually readable designation of both the name and concentration of said associated antimicrobial product carried therein.

32. A kit as claimed in claim 31 wherein said set of test chemicals further comprises a preselected buffer for pH control and a preselected redox stabiliser for lowering the reduction of resazurin by said growth medium and said buffer during incubation of said test panel, and wherein all of the constituents of said set of test chemicals are carried in each of said absorbent paper disks, and said inoculum liquid contains only said microorganism.

33. A kit as claimed in claim 22 comprising at least N test modules each comprising a carrying medium for carrying in dry solid form one of said N different quantities of said antimicrobial product and a test chemical group comprising a subset of the constituents of a set of test chemicals comprising a quantity of resazurin and a quantity of a growth medium for microorganisms; and dispensing means for dispensing said N test modules into said N test wells in an order according to the quantity of said antimicrobial product therein.

34. A kit as claimed in claim 33 comprising a multiplicity of test panels for testing multiple microorganisms and a multiplicity of sets of said N test modules; said dispensing means comprising storage means for storing said multiplicity of sets of N test modules and means for dispensing said test modules one at a time from said storage means, said N test modules in each of said multiplicity of sets being organised in said storage means in a prearranged order according to the quantity of said antimicrobial product therein such that said dispensing means is operable to dispense N test modules in said order into said N test wells in each of said test panels.

35. A kit as claimed in claim 23 wherein said set of test chemicals further comprises a preselected buffer for pH control and wherein all of the constituents of said set of test chemicals are carried in said absorbent paper disk, said inoculum liquid containing only said microorganism.

36. A kit as claimed in claim 34 adapted for testing the quantitative susceptibility of said microorganism to growth inhibition by M different antimicrobial products, wherein
said test panel defines M sets of N test wells, each set being associated with one of said antimicrobial products;
said dispensing means comprises M separate ones of said storage means each associated with one of said M antimicrobial products and having associated means for dispensing N test modules stored therein into associated one of said M set of N test wells;
said inoculator means dispensing said prearranged quantity of said inoculum liquid into each of said N test wells in each of said M sets of test wells in a.sequential one set at a time manner.

## Patentansprüche

1. Verfahren zur Prüfung der Empfindlichkeit eines Mikroorganismus gegenüber Wachstumshemmung durch eine vorgewählte Konzentration eines antimikrobiellen Produkts, umfassend die Schritte:
a. Einbringen einer vorbestimmten Konzentration eines Wachstumsmediums für Mikroorganismen und einer vorbestimmten Konzentration an Resazurin, das vorher in einem Konzentrationsbereich festgesetzt wurde, der durch geringe Toxizität gegenüber Mikroorganismen und beträchtliche Empfindlichkeit gegenüber Reduktion zu Resorufin durch Metaboliten des Mikroorganismus-Wachstums **gekennzeichnet** ist und eines vorgewählten Redox-Stabilisationsmittels, das durch wesentliche Verringerung der Reduktion von Resazurin durch das Wachstumsmedium während Schritt g. **gekennzeichnet** ist, in jeweils ein Aufnahmegefäß zur negativen Wachstumskontrolle und ein Aufnahmegefäß zur positiven Wachstumskontrolle;
b. Einbringen einer vorbestimmten Konzentration des Mikroorganismus in das Aufnahmegefäß zur positiven Wachstumskontrolle;
c. Einbringen der vorgewählten Konzentration des antimikrobiellen Produkts in ein Testaufnahmegefäß;
d. Einbringen der vorbestimmten Konzentration von Resazurin und des vorgewählten Redox-Stabilisationsmittels, das durch wesentliche Verringerung der Reduktion von Resazurin durch das Wachstumsmedium während Schritt g. **gekennzeichnet** ist, in das Testaufnahmegefäß;
e. Einbringen der vorbestimmten Konzentration des Wachstumsmediums in das Testaufnahmegefäß;
f. Einbringen der vorbestimmten Konzentration des Mikroorganismus in das Testaufnahmegefäß;
g. gemeinsames Inkubieren aller Aufnahmegefäße für eine Inkubationszeitdauer, verbunden mit einem vorgewählten Meßprotokoll, umfassend ein Meßprotokoll mit sichtbarem Licht oder ein Meßprotokoll mit Fluoreszenzanregung; und
h. nach der Inkubationszeitdauer das Auswerten der Aufnahmegefäße gemäß dem vorgewählten Meßprotokoll, wobei die Anwesenheit oder Abwesenheit von Wachstum des Mikroorganismus in dem Testaufnahmegefäß auf der Grundlage der relativen Konzentrationen an Resazurin und Resorufin darin bestimmt wird, wobei das Meßprotokoll mit sichtbarem Licht einen Entscheidungalgorithmus enthält, der auf mindestens einer vorbestimmten funktionellen Kombination der in jedem der Aufnahmegefäße detektierten Farbe der sichtbaren Lichtreflexion basiert, wobei das Meßprotokoll mit Fluoreszenzanregung einen Entscheidungsalgorithmus enthält, der auf mindestens einer vorbestimmten funktionellen Kombination der Werte des Fluoreszenzemissionssignals basiert, das durch das Reduktionsprodukt Resorufin in jedem der Aufnahmegefäße erzeugt wird.

2. Verfahren nach Anspruch 1, worin die Schritte c. bis einschließlich f. ausgeführt werden durch
j1) Bilden eines Testmoduls in dem Testaufnahmegefäß, umfassend ein trockenes Feststoffvolumen einer vorbestimmten Menge des antimikrobiellen Produkts und ein trockenes Feststoffvolumen eines vorgewählten Teilsatzes der Bestandteile eines Testchemikaliensatzes, umfassend Resazurin und das Wachstumsmedium; und
j2) Zugeben eines Flüssigkeitsvolumens, worin die vorbestimmte Konzentration des Mikroorganismus und alle nicht in dem Testmodul enthaltenden Bestandteile des Testchemikaliensatzes enthalten sind, in die Testvertiefung, um das Testmodul vor Ausführung des Schrittes g zu rehydratisieren.

3. Verfahren nach Anspruch 2, worin der Testchemikaliensatz einen vorgewählten Puffer zur Kontrolle des endgültigen pH-Wertes der rehydratisierten Testchemikalienlösung und ein vorgewähltes Redox-Stabilisationsmittel enthält, das durch wesentliche Erniedrigung der Reduktion von Resazurin durch das Wachstumsmedium während Schritt g. **gekennzeichnet** ist.

4. Verfahren nach Anspruch 3, worin der Schritt j1) ausgeführt wird durch
k1) Zugeben eines vorbestimmten Flüssigkeitsvolumens, worin die vorgewählte Konzentration des antimikrobiellen Produkts, die vorbestimmte Konzentration an Resazurin, der vorgewählte Puffer, und das vorgewählte Redoxstabilisationsmittel enthalten sind, zu einem Trägermedium,
k2) Trocknen des Trägermediums, um das antimikrobielle Produkt, das Resazurin, den Puffer und das Redoxstabilisationsmittel darin als trockene Feststoffkomponenten zu gewinnen; und
k3) Legen des Trägermediums in das Testaufnahmegefäß entweder bevor oder nachdem die Schritte k1) und k2} durchgeführt werden;
und Schritt j2) ausgeführt wird durch
I1) Zugeben eines Flüssigkeitsvolumens, worin die vorbestimmte Konzentration des Wachstumsmediums und die vorbestimmte Konzentration des Mikroorganismus enthalten sind, in das Testaufnahmegefäß um die trockenen Feststoffkomponenten in dem Trägermedium zu rehydratisieren.

5. Verfahren nach Anspruch 3, worin der Schritt j1) ausgeführt wird durch
k1) Zugeben eines vorbestimmten Flüssigkeitsvolumens, worin die vorgewählte Konzentration des antimikrobiellen Produkts, die vorbestimmte Konzentration des Resazurins, die vorbestimmte Konzentration des Wachstumsmediums, der vorgewählte Puffer und das vorgewählte Redoxstabilisationsmittel enthalten sind, zu einem Trägermedium
k2) Trocknen des Trägermediums, um das antimikrobielle Produkt, das Resazurin, das Wachstumsmedium, den Puffer und das Redoxstabilisationsmittel darin als trockene Feststoffkomponenten zu gewinnen; und
k3) Legen des Trägermediums in das Testaufnahmegefäß entweder bevor oder nachdem die Schritte k1) und k2) durchgeführt werden
und der Schritt j2) ausgeführt wird durch
I1) Zugeben eines Flüssigkeitsvolumens, das die vorbestimmte Konzentration des Mikroorganismus umfasst, in das Testaufnahmegefäß, um die trockenen Feststoffkomponenten in dem Trägermedium zu rehydratisieren.

6. Verfahren nach Anspruch 1, worin der Schritt a. ausgeführt wird durch
j1) Zugeben eines vorbestimmten Flüssigkeitsvolumens worin die vorbestimmte Konzentration des Resazurin, die vorbestimmte Konzentration des Wachstumsmediums, ein vorgewählter Puffer und ein vorgewähltes Redoxstabilisationsmittel enthalten sind, zu jeweils einem Paar an Trägermedien, wobei der vorgewählte Puffer eine vorbestimmte Konzentration zur Kontrolle des pH-Werts der Flüssigkeit enthält;
j2) Trocknen von jedem der Trägermedien, um das Resazurin, das Wachstumsmedium, den Puffer und das Redoxstabilisationsmittel darin als trockene Feststoffkomponenten zu gewinnen;
j3) Legen von jeweils einem der Trägermedien in das Aufnahmegefäß zur negativen Wachstumskontrolle und das Aufnahmegefäß zur positiven Wachstumskontrolle entweder bevor oder nachdem die Schritte j1) und j2) durchgeführt werden; und
j4) Zugeben eines Volumens von steriler Flüssigkeit in das Aufnahmegefäß zur negativen Wachstumskontrolle, um die trockenen Feststoffkomponenten zu rehydratisieren;
wobei die Schritte c., d., und e. ausgeführt werden durch
k1) Zugeben eines vorbestimmten Flüssigkeitsvolumens worin die vorgewählte Konzentration des antimikrobiellen Produkts, die vorbestimmte Konzentration des Resazurin, die vorbestimmte Konzentration an Wachstumsmedium, der vorgewählte Puffer und das vorgewählte Redoxstabilisationsmittel enthalten sind, zu einem Trägermedium;
k2) Trocknen des Trägermediums, um das antimikrobielle Produkt, das Resazurin, das Wachstumsmedium, den Puffer und das Redoxstabilisationsmittel darin als trockene Feststoffkomponenten zu gewinnen; und
k3) Legen des.Trägermediums in das Testaufnahmegefäß entweder bevor oder nachdem die Schritte j1) und j2) durchgeführt werden
und die Schritte b. und f. ausgeführt werden durch
I1) Zugeben eines Flüssigkeitsvolumens, umfassend die vorbestimmte Konzentration des Mikroorganismus in das Aufnahmegefäß zur positiven Wachstumskontrolle und zum Testaufnahmegefäß, um die trockenen Feststoffkomponenten in dem Trägermedium zu rehydratisieren.

7. Verfahren nach Anspruch 1, worin der Schritt g. das gemeinsame Inkubieren der Aufnahmegefäße für eine vorher festgesetzte Inkubationszeitdauer in Verbindung mit einem Meßprotokoll mit Fluoreszenzanregung umfasst;
und der Schritt h. das Auswerten der Aufnahmegefäße gemäß des Meßprotokolls mit Fluoreszenzanregung umfasst, beinhaltend die Schritte
h1) Ablesen des Wertes der Fluoreszenzanregung von Resorufin in jedem der Aufnahmegefäße, wobei der Wert für die Vertiefung zur negativen Wachstumskontrolle als N bezeichnet wird, der Wert für die Vertiefung zur positiven Wachstumskontrolle als P bezeichnet wird und der Wert für das Testaufnahmegefäß als T bezeichnet wird;
h2) Berechnen der Werte eines Wachstumskontrollparameters Gc und eines korrigierten Testparameters Tc als die Differenz der Werte P und N bzw. der Werte T und N;
h3) Berechnen des Wertes einer Testvariablen I, umfassend eine vorbestimmte funktionelle Kombination des Wachstumskontrollparameters Gc und des korrigierten Testparameters Tc; und
h4) Übermitteln eines Testergebnisses unter Verwendung der Testvariablen 1 in einen vorgewählten Entscheidungsalgorithmus.

8. Verfahren nach Anspruch 7, worin
der Schritt h3) das Berechnen der Testvariablen I als eine vorbestimmte Funktion umfasst, welche das Verhältnis von Tc und Gc beinhaltet;
und der Schritt h4) das Übermitteln umfasst von einem positiven Testergebnis (Organismuswachstum), wenn I größer als oder gleich wie ein vorbestimmter positiver Entscheidungswert XP ist,
einem negativen Testergebnis (kein Organismuswachstum), wenn I weniger als oder gleich wie ein vorbestimmter negativer Entscheidungswert XL ist,
und einem unbestimmten Testergebnis, wenn I zwischen XP und XL ist, worin die Entscheidungswerte XL und XP empirisch aus Daten festgesetzt werden, die in Kontrolltests unter Verwendung von Organismen, die bekannte Testergebnisse erzeugen, erhalten werden.

9. Verfahren nach Anspruch 1, worin der Schritt g. gemäß einem schnellen Meßprotokoll mit Fluoreszenzanregung ausgeführt wird und die gemeinsame Inkubation der Aufnahmegefaße für eine Zeitdauer beinhaltet, die ausreicht zur Erzeugung einer vorher festgesetzten Wachstumsrate der Mikroorganismen in dem Aufnahmegefäß zur positiven Wachstumskontrolle wie sie durch Ablesen des Wertes der Fluoreszenzanregung von Resorufin in dem Aufnahmegefäß zur negativen Wachstumskontrolle und dem Aufnahmegefäß zur positiven Wachstumskontrolle zu mindestens zwei verschiedenen Zeiten während der Zeitdauer bestimmt wird;
und der Schritt h. das Auswerten der Aufnahmegefäße gemäß dem schnellen Meßprotokoll mit Fluoreszenzanregung umfasst, welches die Schritte beinhaltet
h1) Bestimmen der Werte von vorbestimmten dynamischen Eigenschaften der Herstellung von Resorufin jeweils im Testaufnahmegefäß und im Aufnahmegefäß zur positiven Wachstumskontrolle durch Messung der Fluoreszenzanregungswerte von Resorufin darin und im Aufnahmgefäß zur negativen Wachstumskontrolle bei mindestens zwei getrennten Zeitabschnitten unter Verwendung der gemessenen Werte des Aufnahmgefäßes zur negativen Wachstumskontrolle zur Korrektur, und Bezeichnen der Werte als T' bzw. G' ;
h2) Berechnen des Werts einer als I bezeichneten Testvariablen, die eine vorbestimmte funktionelle Kombination von T' und G' umfasst; und
h3) Übermitteln eines Testergebnisses unter Verwendung des Werts der Testvariablen I in einen vorgewählten Entscheidungsalgorithmus.

10. Verfahren nach Anspruch 9, worin der Schritt h2) das Berechnen der Testvariable I als eine vorbestimmte Funktion umfasst, welche das Verhältnis von T' und G' beinhaltet;
und Schritt h3) umfasst das Übermitteln von
einem positiven Testergebnis (Organismuswachstum), wenn I größer als oder gleich wie ein vorher festgesetzter positiver Entscheidungswert XP ist,
einem negativen Testergebnis (kein Organismuswachstum), wenn I weniger als oder gleich wie ein vorher festgesetzter negativer Entscheidungswert XL ist, oder
einem unbestimmten Testergebnis, wenn I zwischen XP und XL ist,
worin die Entscheidungswerte XL und XP empirisch aus Daten festgesetzt werden, die in Kontrolltests unter Verwendung von Organismen, die bekannte Testergebnisse erzeugen, erhalten werden.

11. Verfahren nach Anspruch 1, worin der Schritt g. die gemeiname Inkubation der Aufnahmegefäße für eine vorher festgesetzte Inkubationszeitdauer in Verbindung mit einem Meßprotokoll mit sichtbarem Licht umfasst;
und der Schritt h. die Schritte umfasst
h1) Qualifizieren der Aufnahmegefäße zur Auswertung, basierend auf der Detektion einer Abwesenheit einer erkennbaren Verschiebung der anfänglichen blauen Farbe, die Resazurin in dem Aufnahmegefäß zur negativen Wachstumskontrolle zugeordnet werden kann, wodurch kein Wachstum der Mikroorganismen darin angezeigt wird und einer Anwesenheit einer beträchtlichen Farbverschiebung in der Vertiefung zur positiven Wachstumskontrolle von der anfänglichen blauen Farbe des Resazurin zu einer roten Farbe, welche der Erzeugung des Reduktionsprodukts Resorufin durch Mikroorganismuswachstum darin zugeordnet werden kann;
h2) Übermitteln eines mißlungenen Versuchs, wenn die Aufnahmegefäße sich nicht zur Auswertung gemäß Schritt h1) qualifizieren; und
h3) Übermitteln eines Testergebnisses, wenn sich die Aufnahmegefäße zur Auswertung gemäß Schritt i1) qualifizieren durch Detektion der Anwesenheit oder Abwesenheit einer beträchtlichen Farbverschiebung des Testaufnahmegefäßes von einer anfänglichen blauen Farbe des Resazurin zu der roten Farbe des Resorufin, wobei das Testergebnis positiv ist (Organismuswachstum), wenn die Farbverschiebung vorliegt und das Testergebnis negativ ist (kein Organismuswachstum) wenn die Farbverschiebung nicht vorliegt.

12. Verfahren nach Anspruch 3, worin der Schritt j1) ausgeführt wird durch
k1) Zugeben eines vorbestimmten Flüssigkeitsvolumens, worin die vorgewählte Konzentration des antimikroblellen Produkts, die vorbestimmte Konzentration des Resazurin, die vorbestimmte Konzentration des Wachstumsmediums, der vorgewählte Puffer und das vorgewählte Redoxstabilisationsmittel enthalten sind, in das Testaufnahmegefäß; und
k2) Trocknen des Flüssigkeitsvolumens, um das antimikrobielle Produkt, das Resazurin, das Wachstumsmedium, den Puffer und das Redoxstabilisationsmittel als trockene Feststoffkomponenten zu gewinnen;
und der Schritt j2) ausgeführt wird durch
I1) Zugeben eines Flüssigkeitsvolumens, welches die vorbestimmte Konzentration des Mikroorganismus umfasst, in das Testaufnahmegefäß, um die trockenen Feststoffkomponenten zu rehydratisieren.

13. Verfahren nach Anspruch 5, worin das Trägermedium eine absorbierende Papierscheibe umfasst und die trockenen Feststoffkomponenten in der Scheibe gewonnen werden.

14. Verfahren nach Anspruch 5, worin das Trägermedium mindestens eine erste und eine zweite absorbierende Papierscheibe umfasst, wobei die erste Scheibe darin Resazurin enthält, die zweite Scheibe das Wachstumsmedium darin enthält und der Puffer und das Redoxstabilisationsmittel zu einer der ersten oder zeiten Scheiben zugegeben werden.

15. Verfahren nach Anspruch 5, worin der Schritt g. die gemeinsame Inkubation der Aufnahmegefäße für eine vorher festgesetzte Inkubationszeitdauer in Verbindung mit einem Meßprotokoll mit sichtbarem Licht umfasst; und der Schritt h. die Schritte umfasst
h1) Qualifizieren der Aufnahmegefäße zur Auswertung, basierend auf der Detektion einer Abwesenheit einer erkennbaren Verschiebung der anfänglichen blauen Farbe, welche Resazurin in dem Aufnahmegefäß zur negativen Wachstumskontrolle zugeordnet werden kann, wodurch kein Wachstum des Mikroorganismus darin angezeigt wird und einer Anwesenheit einer beträchtlichen Farbverschiebung in der Vertiefung der positiven Wachstumskontrolle von einer anfänglichen blauen Farbe des Resazurin zu einer roten Farbe, welche der Erzeugung des Reduktionsprodukts Resorufin durch Mikroorganismuswachstum darin zugeordnet werden kann;
h2) Übermitteln eines mißlungenen Versuches, wenn die Aufnahmegefäße sich nicht zur Auswertung gemäß Schritt h1) qualifizieren; und
h3) Übermitteln eines Testergebnisses, wenn sich die Aufnahmegefäße zur Auswertung gemäß Schritt i1) qualifizieren durch Detektion der Anwesenheit oder Abwesenheit einer beträchtlichen Farbverschiebung des Testaufnahmegefäßes von einer anfänglichen blauen Farbe des Resazurin zu der roten Farbe des Resorufin, wobei das Testergebnis positiv (Organismuswachstum) ist, wenn die Farbverschiebung vorliegt und das Testergebnis negativ (kein Organismuswachstum) ist, wenn die Farbverschiebung nicht vorliegt.

16. Vorrichtung zur Prüfung der qualitativen Empfindlichkeit eines Mikroorganismus gegenüber Wachstumshemmung durch ein antimikrobielles Produkt unter Verwendung eines vorbestimmten qualitativen Empfindlichkeitstestprotokolls, beinhaltend erste und zweite Mengen des antimikrobiellen Produkts, umfassend eine Testplatte, bestimmend eine Vertiefung zur negativen Wachstumskontrolle, eine Vertiefung zur positiven Wachstumskontrolle und ein Paar Testvertiefungen;
ein Testmodul, jeweils in die Vertiefung zur negativen Wachstumskontrolle, in die Vertiefung zur positiven Wachstumskontrolle und in das Testvertiefungspaar eingebracht, wobei jedes der Testmodule ein trockenes Feststoffvolumen eines Teilsatzes der Bestandteile eines Testchemikaliensatzes umfasst, umfassend eine Menge Resazurin und eine Menge Wachstumsmedium für Mikroorganismen und ein vorgewähltes Redox-Stabilisationsmittel, das durch wesentliche Verringerung der Reduktion von Resazurin durch das Wachstumsmedium **gekennzeichnet** ist, wobei jedes der Testmodule in dem Testvertiefungspaar weiterhin ein trockenes Feststoffvolumen der ersten oder zweiten Menge des antimikrobiellen Produkts umfasst;
wobei das Testmodul in der Vertiefung zur negativen Wachstumskontrolle eingerichtet ist zur Rehydratisierung durch ein Volumen von steriler Flüssigkeit, enthaltend alle Bestandteile des Testchemikaliensatzes, die nicht in dem Teilsatz enthalten sind;
wobei die Testmodule jeweils in der Vertiefung zur positiven Wachstumskontrolle und dem Testvertiefungspaar eingerichtet sind zur Rehydratisierung durch ein Volumen von Inokulations-Flüssigkeit, enthaltend alle Bestandteile des Testchemikaliensatzes, die nicht in dem Teilsatz enthalten sind, zusammen mit einer vorbestimmten Konzentration des Mikroorganismus;
wobei das Volumen von steriler Flüssigkeit und das Volumen von Inokulations-Flüssigkeit die gleiche vorgewählte Volumenmenge aufweisen, wobei die Menge an Resazurin relativ zu der vorgewählten Volumenmenge vorgewählt wird, um eine vorgewählte Konzentration von Resazurin in jeder Vertiefung in einem Bereich zu erzeugen, der durch niedrige Toxizität gegenüber Mikroorganismuswachstum und beträchtliche Empfindlichkeit gegenüber Reduktion zu Resorufin durch Metaboliten des Mikroorganismuswachstums **gekennzeichnet** ist und die Menge an Wachstumsmedium vorgewählt ist, um eine vorgewählte Konzentration an Wachstumsmedium in jeder der Vertiefungen zu erzeugen;
wobei die Testplatte und die darin befindlichen rehydratisierten Testmodule eingerichtet sind, um für eine Inkubationszeitdauer inkubiert zu werden in Verbindung mit einem vorgewählten Meßprotokoll umfassend ein Meßprotokoll mit sichtbarem Licht oder ein Meßprotokoll mit Fluoreszenzanregung und danach zur Auswertung gemäß dem vorgewählten Meßprotokoll, um die Anwesenheit oder Abwesenheit von Wachstum der Mikroorganismen in den Testvertiefungen zu bestimmen.

17. Vorrichtung zur Prüfung der quantitativen Empfindlichkeit eines Mikroorganismus gegenüber Wachstumshemmung durch ein antimikrobielles Produkt unter Verwendung eines vorbestimmten quantitativen Empfindlichkeitstestprotokolls, beinhaltend N unterschiedliche Mengen des antimikrobiellen Produkts, worin N>2, umfassend
eine Testplatte, welche eine Vertiefung zur negativen Wachstumskontrolle, eine Vertiefung zur positiven Wachstumskontrolle und N Testvertiefungen bestimmt,
ein Testmodul, eingebracht jeweils in die Vertiefung zur negativen Wachstumskontrolle, in die Vertiefung zur positiven Wachstumskontrolle und in die N Testvertiefungen, wobei jedes Testmodul ein trockenes Feststoffvolumen eines Teilsatzes der Bestandteile eines Testchemikaliensatzes enthält, umfassend eine Menge Resazurin und eine Menge von Wachstumsmedium für Mikroorganismen und ein vorgewähltes Redox-Stabilisationsmittel, das durch wesentliche Verringerung der Reduktion von Resazurin durch das Wachstumsmedium **gekennzeichnet** ist, wobei jedes der Testmodule in den N Testvertiefungen weiterhin ein trockenes Feststoffvolumen einer unterschiedlichen Menge des antimikrobiellen Produkts, ausgewählt gemäß dem quantitativen Empfindlichkeitstestprotokoll, enthält;
wobei das Testmodul in der Vertiefung zur negativen Wachstumskontrolle eingerichtet ist zur Rehydratisierung mit einem Volumen von steriler Flüssigkeit, enthaltend alle Bestandteile des Testchemikaliensatzes, die nicht in dem Teilsatz enthalten sind;
wobei die Testmodule in jeweils der Vertiefung zur positiven Wachstumskontrolle und in den N Testvertiefungen eingerichtet sind zur Rehydratisierung mit einem Volumen von Inokulations-Flüssigkeit, enthaltend alle Bestandteile des Testchemikaliensatzes, die nicht in dem Teilsatz enthalten sind, zusammen mit einer vorbestimmten Konzentration des Mikroorganismus;
wobei das Volumen von steriler Flüssigkeit und das Volumen von Inokulations-Flüssigkeit die gleiche vorgewählte Volumenmenge aufweisen, wobei die Menge von Resazurin relativ zu der vorgewählten Volumenmenge ausgewählt wird, um eine vorgewählte Konzentration von Resazurin in jeder der Vertiefungen zu erzeugen in einem Bereich, der durch niedrige Toxizität gegenüber Mikroorganismuswachstum und beträchtliche Empfindlichkeit gegenüber Reduktion zu Resorufin durch Metaboliten des Mikroorganismuswachstums **gekennzeichnet** ist und die Menge an Wachstumsmedium ausgewählt ist, um eine vorgewählte Konzentration an Wachstumsmedium in jeder der Vertiefungen zu erzeugen;
wobei die Testplatte und die darin enthaltenden rehydratisierten Testmodule eingerichtet sind zur Inkubation für eine Inkubationszeitdauer in Verbindung mit einem vorgewählten Meßprotokoll, umfassend ein Meßprotokoll mit sichtbarem Licht oder ein Meßprotokoll mit Fluoreszenzanregung und danach zur Auswertung gemäß dem vorgewählten Meßprotokoll, um die Anwesenheit oder Abwesenheit von Wachstum des Mikroorganismus in den Testvertiefungen zu bestimmen.

18. Vorrichtung nach Anspruch 16 oder Anspruch 17, worin jedes der Testmodule ein Trägermedium, enthalten in einer entsprechenden Vertiefung umfasst und jedes der Testmodule den Teilsatz der Bestandteile des Testchemikaliensatzes enthält.

19. Vorrichtung nach Anspruch 18, worin das Trägermedium eine absorbierende Papierscheibe umfasst und jede der absorbierenden Papierscheiben in den Testvertiefungen mit einer sichtbar lesbaren Anzeige sowohl des Namens als auch der Konzentration des darin aufbewahrten antimikrobiellen Produkts bedruckt ist.

20. Vorrichtung nach Anspruch 18, worin das Trägermedium mindestens eine erste und eine zweite absorbierende Papierscheibe umfasst, wobei die erste Papierscheibe das Resazurin enthält und die zweite Papierscheibe das Wachstumsmedium enthält, wobei jeweils eine der beiden Papierscheiben in den Testvertiefungen die entsprechende Menge des antimikrobiellen Produkts trägt und mit einer sichtbar lesbaren Anzeige des Namens und der Konzentration des darin aufbewahrten antimikrobiellen Produkts bedruckt ist.

21. Kit zur Prüfung der qualitativen Empfindlichkeit eines Mikroorganismus gegenüber Wachstumshemmung durch ein antimikrobielles Produkt unter Verwendung eines vorbestimmten qualitativen Empfindlichkeitstestprotokolls, beinhaltend eine erste und zweite Menge des antimikrobiellen Produkts, umfassend
eine Testplatte, bestimmend eine Vertiefung zur negativen Wachstumskontrolle, eine Vertiefung zur positiven Wachstumskontrolle und ein Paar von Testvertiefungen;
ein Testmodul, enthalten jeweils in der Vertiefung zur negativen Wachstumskontrolle, in der Vertiefung zur positiven Wachstumskontrolle und in dem Paar von Testvertiefungen, wobei die Testmodule jeweils ein trockenes Feststoffvolumen eines Teilsatzes der Bestandteile eines Testchemikaliensatzes enthalten, umfassend eine Menge Resazurin und eine Menge Wachstumsmedium für Mikroorganismen und ein vorgewähltes Redox-Stabilisationsmittel, das durch wesentliche Verringerung der Reduktion von Resazurin durch das Wachstumsmedium **gekennzeichnet** ist, wobei die Testmodule in dem Paar von Testvertiefungen jeweils weiterhin ein trockenes Feststoffvolumen von einer ersten oder zweiten Menge des antimikrobiellen Produkts enthalten;
einen Behälter mit Rehydratisierungs-Flüssigkeit, enthaltend alle Bestandteile des Testchemikaliensatzes, die nicht in dem Teilsatz enthalten sind;
Inokulum-Aufbewahrungsmittel zur Aufbewahrung eines Volumens von Rehydratisierungs-Flüssigkeit, zu dem der Mikroorganismus zugegeben wurde, um eine Inokulations-Flüssigkeit zu bilden mit einer vorbestimmten Konzentration an Mikroorganismen darin;
Inokulationsmittel, beinhaltend Mittel zur Abgabe einer vorbestimmten Menge der Rehydratisierungs-Flüssigkeit in die Vertiefung zur negativen Wachstumskontrolle, Mittel zur Abgabe einer vorbestimmten Menge der InokulationsFlüssigkeit in die Vertiefung zur positiven Wachstumskontrolle und das Paar von Testvertiefungen, um die Testchemikalien in den Testmodulen darin zu rehydratisieren;
wobei die Menge von Resazurin relativ zu der vorbestimmten Menge vorgewählt ist, um eine vorgewählte Konzentration an Resazurin in jeder der Vertiefungen in einem Bereich zu erzeugen, der durch niedrige Toxizität gegenüber Mikroorganismuswachstum und beträchtliche Empfindlichkeit gegenüber Reduktion zu Resorufin durch Metaboliten des Mikroorganismuswachstums **gekennzeichnet** ist und die Menge des Wachstumsmediums vorgewählt ist, um eine vorgewählte Konzentration von Standardwachstumsmedium in jeder der Vertiefungen zu erzeugen;
wobei die Testplatte und die darin enthaltenen rehydratisierten Testmodule eingerichtet sind zur Inkubation für eine Inkubationszeitdauer in Verbindung mit einem vorgewählten Meßprotokoll, umfassend ein Meßprotokoll mit sichbarem Licht oder ein Meßprotokoll mit Fluoreszenzanregung und danach zur Auswertung gemäß dem vorgewählten Meßprotokoll, um die Anwesenheit oder Abwesenheit von Wachstum der Mikroorganismen in den Testvertiefungen zu bestimmen.

22. Kit zur Prüfung der quantitativen Empfindlichkeit eines Mikroorganismus gegenüber Wachstumshemmung durch ein antimikrobielles Produkt unter Verwendung eines vorbestimmten quantitativen Empfindlichkeitstestprotokolls, beinhaltend N unterschiedliche Mengen des antimikrobiellen Produkts, worin N>2, umfassend eine Testplatte, bestimmend eine Vertiefung zur negativen Wachstumskontrolle, eine Vertiefung zur positiven Wachstumskontrolle und N Testvertiefungen;
ein Testmodul, enthalten jeweils in der Vertiefung zur negativen Wachstumskontrolle, in der Vertiefung zur positiven Wachstumskontrolle und in den N Testvertiefungen, wobei die Testmodule jeweils ein trockenes Feststoffvolumen eines Teilsatzes der Bestandteile eines Testchemikaliensatzes enthalten, umfassend eine Menge an Resazurin und eine Menge an Wachstumsmedium für Mikroorganismen und ein vorgewähltes Redox-Stabilisationsmittel, das durch wesentliche Verringerung der Reduktion von Resazurin durch das Wachstumsmedium **gekennzeichnet** ist, wobei die Testmodule in den N Testvertiefungen jeweils weiterhin ein trockenes Feststoffvolumen von einer von N unterschiedlichen Mengen des antimikrobiellen Produkts umfassen;
einen Behälter mit Rehydratisierungs-Flüssigkeit, enthaltend alle Bestandteile des Testchemikaliensatzes, die nicht in dem Teilsatz enthalten sind;
Inokulum-Aufbewahrungsmittel zur Aufbewahrung eines Volumens von Rehydratisierungs-Flüssigkeit mit dazu zugegebenen Mikroorganismen, um eine Inokulations-Flüssigkeit zu bilden, die eine vorbestimmte Konzentration an Mikroorganismus darin enthält;
Inokulations-Mittel, beinhaltend Mittel zur Abgabe einer vorbestimmten Menge der Rehydratisierungs-Flüssigkeit in die Vertiefung zur negativen Wachstumskontrolle, Mittel zur Abgabe einer vorbestimmten Menge der Inokulations-Flüssigkeit in die Vertiefung zur positiven Wachstumskontrolle und jeweils in die N Testvertiefungen, um die Testchemikalien in den Testmodulen darin zu rehydratisieren;
wobei die Menge an Resazurin vorgewählt ist, relativ zu der vorbestimmten Menge, um eine vorgewählte Konzentration an Resazurin in jeder der Vertiefungen zu erzeugen, in einem Bereich, der durch niedrige Toxizität gegenüber Mikroorganismuswachstum und beträchtliche Empfindlichkeit gegenüber Reduktion zu Resorufin durch Metabolite des Mikroorganismuswachstums **gekennzeichnet** ist, und die Menge des Wachstumsmediums vorgewählt ist, um eine vorgewählte Konzentration von Standardwachstumsmedium in jeder der Vertiefungen zu erzeugen;
wobei die Testplatte und die darin enthaltenen rehydratisierten Testmodule eingerichtet sind zur Inkubation für eine Inkubationszeitdauer in Verbindung mit einem vorgewählten Meßprotokoll, umfassend ein Meßprotokoll mit sichtbarem Licht oder ein Meßprotokoll mit Fluoreszenzanregung und danach zur Auswertung gemäß dem vorgewählten Meßprotokoll, um die Anwesenheit oder die Abwesenheit von wachstum des Mikroorganismus in den Testvertiefungen zu bestimmen.

23. Kit nach Anspruch 21 oder Anspruch 22, worin die Testmodule jeweils eine absorbierende Papierscheibe umfassen und jede der absorbierenden Papierscheiben in den Testvertiefungen mit einer sichtbar lesbaren Anzeige des Namens und der Konzentration des darin aufbewahrten mikrobiellen Produkts bedruckt ist.

24. Kit nach Anspruch 21 oder Anspruch 22, worin jedes der Testmodule mindestens eine erste und eine zweite absorbierende Papierscheibe umfasst, wobei die erste Scheibe das Resazurin enthält und die zweite Scheibe das Wachstumsmedium enthält, wobei eine der ersten und zweiten Papierscheiben in jeder der Testvertiefungen, die entsprechende Menge des antimikrobiellen Produkts enthält und jeweils mit einer sichtbar lesbaren Anzeige des Namens und der Konzentration des darin aufbewahrten antimikrobiellen Produkts bedruckt ist.

25. Kit umfassend ein erstes Testmodul nach Anspruch 21,
ein zweites Testmodul, umfassend ein Trägermedium zum Tragen einer zweiten Menge des antimikrobiellen Produkts in trockener Feststofform und die Testchemikaliengruppe; und
Abgabemittel zur Abgabe des ersten und zweiten Testmoduls in das Paar von Testvertiefungen.

26. Kit nach Anspruch 25, zusätzlich umfassend
ein Paar Wachstumstestmodule, jeweils umfassend ein Trägermedium zum Tragen der Testchemikaliengruppe in trockener Feststofform; und
einen Behälter der Rehydratisierungs-Flüssigkeit;
wobei das Abgabemittel Mittel zur Abgabe von einem der Paare von Wachstumstestmodulen, jeweils in die Vertiefung zur negativen Wachstumskontrolle und die Vertiefung zur positiven Wachstumskontrolle, enthält; und
das Inokulations-Mittel Mittel zur Abgabe einer vorbestimmten Menge der Rehydratisierungs-Flüssigkeit in die Vertiefung zur negativen Wachstumskontrolle und einer vorbestimmten Menge der Inokulations-Flüssigkeit in die Vertiefung zur positiven Wachstumskontrolle enthält.

27. Kit nach Anspruch 25, umfassend eine Vielzahl an Testplatten zur Prüfung von einer Vielzahl von Mikroorganismen und eine Vielzahl von jeweils ersten Testmodulen und zweiten Testmodulen; und
wobei die Abgabemittel erste und zweite Aufbewahrungsmittel zur Aufbewahrung der Vielzahl der ersten bzw. zweiten Testmodule und ein Abgabegerät umfassen, operativ verbunden mit den Aufbewahrungsmitteln zur gleichzeitigen Abgabe von einem der ersten Testmodule aus den ersten Aufbewahrungsmitteln in eine der Testvertiefungen und eines der zweiten Testmodule aus den zweiten Aufbewahrungsmitteln in die andere der Testvertiefungen in einer Testplatte aus der Vielzahl an Testplatten.

28. Kit nach Anspruch 25, umfassend eine Vielzahl an Testplatten zur Prüfung einer Vielzahl von Mikroorganismen und eine Vielzahl von jeweils ersten Testmodulen und zweiten Testmodulen;
wobei die Abgabemittel Aufbewahrungsmittel zur Aufbewahrung der Vielzahl der ersten und zweiten Testmodule in einer abwechselnden Art und ein Abgabegerät umfassen, operativ verbunden mit den Aufbewahrungsmitteln zur alternierenden Abgabe eines ersten Testmoduls in eine der Testvertiefungen in einer der Testplatten und eines zweiten Testmoduls in die andere der Testvertiefungen derselben Testplatte.

29. Kit nach Anspruch 23, worin der Testchemikaliensatz weiterhin einen vorgewählten Puffer zur pH-Kontrolle umfasst und worin alle Bestandteile des Testchemikaliensatzes in der absorbierenden Papierscheibe enthalten sind, wobei die Inokulations-Flüssigkeit nur den Mikroorganismus enthält.

30. Kit nach Anspruch 25, eingerichtet zur Prüfung der qualitativen Empfindlichkeit des Mikroorganismus gegenüber Wachstumshemmung durch M unterschiedliche antimikrobielle Produkte, worin
die Testplatte M Paare an Testvertiefungen festlegt, wobei jedes Paar mit einem der M antimikrobiellen Produkte assoziiert ist;
M erste Testmodule und M zweite Testmodule bereitgestellt werden, die jeweils mit einem der antimikrobiellen Produkte assoziiert sind;
wobei die Abgabemittel Mittel zur Abgabe von jeweils M ersten Testmodulen und jeweils M zweiten Testmodulen in die entsprechenden der M Paare von Testvertiefungen umfassen; und
die Inokulationsmittel eine vorbestimmte Menge der Inokulations-Flüssigkeit in jede Vertiefung der M Paare von Testvertiefungen abgeben.

31. Kit nach Anspruch 30, worin jedes der M ersten und zweiten Testmodule eine absorbierende Papierscheibe umfasst, welche mit einer sichtbar lesbaren Bezeichnung sowohl des Namens als auch der Konzentration des assoziierten darin enthaltenen antimikrobiellen Produkts bedruckt ist.

32. Kit nach Anspruch 31, worin der Testchemikaliensatz weiterhin einen vorgewählten Puffer zur pH-Kontrolle und ein vorgewähltes Redoxstabilisationsmittel zur Erniedrigung der Reduktion von Resazurin durch das Wachstumsmedium und den Puffer während der Inkubation der Testplatte, umfasst und worin alle Bestandteile des Testchemikaliensatzes in jeder der absorbierenden Papierscheiben enthalten sind und die Inokulations-Flüssigkeit nur den Mikroorganismus enthält.

33. Kit nach Anspruch 22, umfassend mindestens N Testmodule, jeweils enthaltend ein Trägermedium zum Tragen einer von N unterschiedlichen Mengen des antimikrobiellen Produkts in trockener Feststofform und eine Testchemikaliengruppe, umfassend einen Teilsatz der Bestandteile des Testchemikaliensatzes, umfassend eine Menge an Resazurin und eine Menge an Wachstumsmedium für Mikroorganismen; und
Abgabemittel zur Abgabe der N Testmodule in die N Testvertiefungen gemäß der Reihenfolge der Menge des darin befindlichen antimikrobiellen Produkts.

34. Kit nach Anspruch 33, umfassend eine Vielzahl an Testplatten zur Prüfung einer Vielzahl von Mikroorganismen und eine Vielzahl an Sätzen der N Testmodule;
wobei die Abgabemittel Aufbewahrungsmittel zur Aufbewahrung der Vielzahl der Sätze an N Testmodulen und Mittel zur einzelnen Abgabe der Testmodule von den Aufbewahrungsmitteln umfassen, wobei die N Testmodule in jeder der Vielzahl der Sätze in den Aufbewahrungsmitteln in einer vorbestimmten Reihenfolge gemäß der Menge des darin enthaltenen antimikrobiellen Produkts vorbestimmt sind, so daß die Abgabemittel N Testmodule in der Reihenfolge in die N Testvertiefungen in jede der Testplatte betriebsmäßig abgeben können.

35. Kit nach Anspruch 23, worin der Testchemikaliensatz weiterhin einen vorgewählten Puffer zur pH-Kontrolle umfasst und worin alle Bestandteile des Testchemikaliensatzes in der absorbierenden Papierscheibe enthalten sind, wobei
die Inokulations-Flüssigkeit nur den Mikroorganismus enthält.

36. Kit nach Anspruch 34, eingerichtet zur Prüfung der quantitativen Empfindlichkeit des Mikroorganismus gegenüber Wachstumshemmung durch M unterschiedliche antimikrobielle Produkte, worin
die Testplatte M Sätze an N Testvertiefungen festlegt, wobei jeder Satz mit einem der antimikrobiellen Produkte assoziiert ist;
die Abgabemittel M separate Aufbewahrungsmittel umfassen, wobei jedes mit einem der M antimikrobiellen Produkte assoziiert ist und begleitende Mittel zur Abgabe von N darin aufbewahrten Testmodulen in den assoziierten der M Sätze von N Testvertiefungen umfasst;
die Inokulations-Mittel die vorbestimmte Menge der Inokulations-Flüssigkeit in jede der N Testvertiefungen in jeden der M Sätze von Testvertiefungen in einen folgenden Satz zeitlich abgeben.

## Revendications

1. Procédé pour tester la sensibilité d'un micro-organisme à une inhibition de croissance par une concentration, choisie préalablement, d'un produit antimicrobien, comprenant les étapes consistant :
(a) à placer dans chacun des réceptacles consistant en un réceptacle témoin de croissance négative et un réceptacle témoin de croissance positive, une concentration, ajustée préalablement, d'un milieu de croissance pour micro-organismes et une concentration, ajustée préalablement, de résazurine prédéterminée de manière à être située dans une plage de concentrations **caractérisée par** une faible toxicité vis-à-vis des micro-organismes et une sensibilité importante à la réduction en résorufine par les produits métaboliques de croissance des micro-organismes, et un stabilisant redox, choisi préalablement, **caractérisé par** une diminution substantielle de la réduction de la résazurine par ledit milieu de croissance au cours de l'étape (g) ;
(b) à placer dans ledit réceptacle témoin de croissance positive une concentration, ajustée préalablement, dudit micro-organisme ;
(c) à placer dans un réceptacle d'essai ladite concentration, choisie préalablement, dudit produit anti-microbien ;
(d) à placer dans ledit réceptacle d'essai ladite concentration, ajustée préalablement, de résazurine, et un stabilisant redox, choisi préalablement, **caractérisé par** une diminution substantielle de la réduction de la résazurine par ledit milieu de croissance au cours de l'étape (g) ;
(e) à placer dans ledit réceptacle d'essai ladite concentration, ajustée préalablement, de milieu de croissance ;
(f) à placer dans ledit réceptacle d'essai ladite concentration, ajustée préalablement, dudit micro-organisme ;
(g) à mettre conjointement en incubation l'ensemble desdits réceptacles pendant un temps d'incubation associé à un protocole de lecture, choisi préalablement, comprenant un des protocoles consistant en un protocole de lecture de lumière visible et un protocole de lecture d'excitation de fluorescence ; et
(h) après ledit temps d'incubation, à soumettre à une lecture lesdits réceptacles en fonction dudit protocole de lecture choisi préalablement pour déterminer la présence ou l'absence de croissance dudit micro-organisme dans ledit réceptacle d'essai sur la base des concentrations relatives de résazurine et de résorufine présentes, ladite lecture de lumière visible comprenant un algorithme de décision basé sur au moins une association fonctionnelle prédéterminée de la couleur de réflectance de la lumière visible détectée dans chacun desdits réceptacles, ledit protocole de lecture d'excitation de fluorescence comprenant un algorithme de décision basé sur au moins une association fonctionnelle prédéterminée des valeurs du signal d'émission de fluorescence engendré par le produit de réduction consistant en résorufine dans chacun desdits réceptacles.

2. Procédé suivant la revendication 1, dans lequel lesdites étapes c. à f. sont mises en oeuvre :
j1) en formant dans ledit réceptacle d'essai un module d'essai comprenant un volume solide sec d'une quantité, ajustée préalablement, dudit produit antimicrobien et un volume solide sec d'une sous-catégorie, choisie préalablement, des constituants d'une série d'agents chimiques d'essai comprenant ladite résazurine et ledit milieu de croissance ; et
j2) en introduisant dans ledit puits d'essai un volume de liquide renfermant la concentration, ajustée préalablement, dudit micro-organisme et tous les constituants de ladite série d'agents chimiques d'essai non présents dans ledit module d'essai pour réhydrater ledit module d'essai avant la mise en oeuvre de ladite étape (g).

3. Procédé suivant la revendication 2, dans lequel la série d'agents chimiques d'essai comprend un tampon choisi préalablement pour l'ajustement du pH final de la solution chimique d'essai réhydratée et un stabilisant redox, choisi préalablement, **caractérisé par** une diminution importante de la réduction de résazurine par ledit milieu de croissance au cours de l'étape (g).

4. Procédé suivant la revendication 3, dans lequel l'étape j1) est mise en oeuvre
k1) en distribuant sur un milieu de support un volume, ajusté préalablement, de liquide renfermant ladite concentration choisie préalablement dudit produit antimicrobien, ladite concentration ajustée préalablement de résazurine, ledit tampon choisi préalablement et ledit stabilisant redox choisi préalablement ;
k2) en séchant ledit milieu de support pour capturer ledit produit antimicrobien, ladite résazurine, ledit tampon et ledit stabilisant redox qui s'y trouvent, sous forme de constituants solides secs ; et
k3) en plaçant ledit milieu de support dans ledit réceptacle d'essai avant ou après la mise en oeuvre desdites étapes k1) et k2)
et ladite étape j2) est mise en oeuvre
11) en introduisant dans ledit réceptacle d'essai un volume de liquide renfermant ladite concentration, ajustée préalablement, dudit milieu de croissance et ladite concentration, ajustée préalablement, dudit micro-organisme pour réhydrater lesdits constituants solides secs dans ledit milieu de support.

5. Procédé suivant la revendication 3, dans lequel ladite étape j1) est mise en oeuvre
k1) en distribuant sur un milieu de support un volume, ajusté préalablement, de liquide renfermant ladite concentration choisie préalablement dudit produit antimicrobien, ladite concentration ajustée préalablement de ladite résazurine, ladite concentration ajustée préalablement dudit milieu de croissance, ledit tampon choisi préalablement et ledit stabilisant redox choisi préalablement ;
k2) en séchant ledit milieu de support pour capturer ledit produit antimicrobien, ladite résazurine, ledit tampon et ledit stabilisant redox qui s'y trouvent, sous forme de constituants solides secs ; et
k3) en plaçant ledit milieu de support dans ledit réceptacle d'essai avant ou après la mise en oeuvre desdites étapes k1) et k2) ;
et ladite étape j2) est mise en oeuvre
11) en introduisant dans ledit réceptacle d'essai un volume de liquide comprenant ladite concentration, ajustée préalablement, dudit micro-organisme pour réhydrater lesdits constituants solides secs dans ledit milieu de support.

6. Procédé suivant la revendication 1, dans lequel ladite étape (a) est mise en oeuvre
j1) en distribuant sur chacun d'une paire de milieux de support un volume, ajusté préalablement, de liquide renfermant ladite concentration ajustée préalablement de ladite résazurine, ladite concentration ajustée préalablement dudit milieu de croissance, un tampon choisi préalablement et un stabilisant redox choisi préalablement, ledit tampon choisi préalablement ayant une concentration ajustée préalablement pour la régulation du pH dudit liquide ;
j2) en séchant chacun desdits milieux de support pour capturer ladite résazurine, ledit milieu de croissance, ledit tampon et ledit stabilisant redox qui s'y trouvent, sous forme de constituants solides secs ;
j3) en plaçant un desdits milieux de support dans chacun des réceptacles consistant en ledit réceptacle témoin de croissance négative et ledit réceptacle témoin de croissance positive avant ou après la mise en oeuvre desdites étapes j1) et j2) ; et
j4) en introduisant dans ledit réceptacle témoin de croissance négative un volume de liquide stérile pour réhydrater lesdits constituants solides secs;
et les étapes (c), (d) et (e) sont mises en oeuvre
k1) en distribuant sur un milieu de support un volume, ajusté préalablement, de liquide renfermant ladite concentration choisie préalablement dudit produit antimicrobien, ladite concentration ajustée préalablement de ladite résazurine, ladite concentration ajustée préalablement dudit milieu de croissance, ledit tampon choisi préalablement et ledit stabilisant redox choisi préalablement ;
k2) en séchant ledit milieu de support pour capturer ledit produit antimicrobien, ladite résazurine, ledit milieu de croissance, ledit tampon et ledit stabilisant redox qui s'y trouvent, sous forme de constituants solides secs ; et
k3) en plaçant ledit milieu de support dans ledit réceptacle d'essai avant ou après la mise en oeuvre desdites étapes j1) et j2) ;
et lesdites étapes (b) et (f) sont mises en oeuvre
l1) en introduisant dans ledit réceptacle témoin de croissance positive et ledit réceptacle d'essai un volume de liquide comprenant ladite concentration, ajustée préalablement, dudit micro-organisme pour réhydrater lesdits constituants solides secs dans ledit milieu de support.

7. Procédé suivant la revendication 1, dans lequel
ladite étape (g) comprend la mise en incubation desdits réceptacles conjointement pendant un temps d'incubation prédéterminé associé à un protocole de lecture d'excitation de fluorescence ;
et ladite étape (h) consiste à effectuer une lecture desdits réceptacles conformément audit protocole de lecture d'excitation de fluorescence, comprenant les étapes
h1) de lecture de la valeur d'excitation de fluorescence de la résorufine dans chacun desdits réceptacles, ladite valeur pour ledit puits témoin de croissance négative étant désignée par N, ladite valeur pour ledit puits témoin de croissance positive étant désignée par P et ladite valeur pour ledit réceptacle d'essai étant désignée par T ;
h2) de calcul des valeurs d'un paramètre témoin de croissance Gc et d'un paramètre d'essai corrigé Tc respectivement en tant que différence entre les valeurs P et N et les valeurs T et N ;
h3) de calcul de la valeur d'une variable d'essai I comprenant une combinaison fonctionnelle conçue préalablement dudit paramètre témoin de croissance Gc et dudit paramètre d'essai corrigé Tc ; et
h4) de report d'un résultat d'essai utilisant ladite variable d'essai I dans un algorithme de décision choisi préalablement.

8. Procédé suivant la revendication 7, dans lequel ladite étape h3) comprend le calcul de ladite variable d'essai I sous forme d'une fonction conçue préalablement qui comprend le rapport de Tc et Gc ;
et ladite étape h4) comprend l'indication d'un des résultats consistant en
un résultat d'essai positif (croissance de l'organisme) si I est supérieure ou égale à une valeur de décision positive prédéterminée XP,
un résultat d'essai négatif (absence de croissance de l'organisme) si I est inférieure ou égale à une valeur de décision négative prédéterminée XL, et
un résultat d'essai indéterminé si I est située entre XP et XL,
lesdites valeurs de décision XL et XP étant déterminées empiriquement à partir des résultats obtenus dans des essais contrôlés au moyen d'organismes qui engendrent des résultats d'essai connus.

9. Procédé suivant la revendication 1, dans lequel ladite étape (g) est mise en oeuvre conformément à un protocole de lecture d'excitation de fluorescence rapide et comprend la mise en incubation desdits réceptacles conjointement pendant un temps suffisant pour produire une vitesse prédéterminée de croissance desdits micro-organismes dans ledit réceptacle témoin de croissance positive, déterminée par la lecture de la valeur d'excitation de fluorescence de la résorufine dans ledit réceptacle témoin de croissance négative et ledit réceptacle témoin de croissance positive à au moins deux moments distincts au cours dudit temps ;
et ladite étape (h) consiste à effectuer une lecture desdits réceptacles conformément audit protocole de lecture d'excitation de fluorescence rapide, qui comprend les étapes
h1) de détermination des valeurs de caractéristiques dynamiques, ajustées préalablement, de production de résorufine dans chacun des réceptacles consistant en ledit réceptacle d'essai et ledit réceptacle témoin de croissance positive par mesure des valeurs d'excitation de fluorescence de la résorufine qui s'y trouve et dans ledit réceptacle témoin de croissance négative à au moins deux temps distincts, en utilisant les valeurs mesurées obtenues à partir dudit réceptacle témoin de croissance négative pour la correction, et en désignant lesdites valeurs respectivement par T' et G' ;
h2) de calcul de la valeur d'une variable d'essai, désignée par I et comprenant une combinaison fonctionnelle conçue préalablement de T' et G' ; et
h3) d'indication d'un résultat d'essai au moyen de la valeur de ladite variable d'essai I dans un algorithme de décision choisi préalablement.

10. Procédé suivant la revendication 9, dans lequel ladite étape h2) comprend le calcul de ladite variable d'essai I en tant qu'une fonction conçue préalablement qui comprend le rapport de T' et G' ;
et ladite étape h3) comprend le report d'un des résultats consistant en
un résultat d'essai positif (croissance de l'organisme) si I est supérieure ou égale à une valeur de décision positive prédéterminée XP,
un résultat d'essai négatif (aucune croissance de l'organisme) si I est inférieure ou égale à une valeur de décision négative prédéterminée XL, et
un résultat d'essai indéterminé si I est comprise entre XP et XL,
lesdites valeurs de décision XL et XP étant déterminées empiriquement à partir des résultats obtenus dans des essais contrôlés au moyen d'organismes qui produisent des résultats d'essai connus.

11. Procédé suivant la revendication 1, dans lequel ladite étape (g) comprend la mise en incubation desdits réceptacles conjointement pendant un temps d'incubation prédéterminé associé à un protocole de lecture de lumière visible ; et ladite étape (h) comprend les étapes
h1) de qualification desdits réceptacles pour la lecture sur la base de la détection de l'absence de variation discernable de la couleur bleue initiale pouvant être attribuée à la résazurine dans ledit réceptacle témoin de croissance négative, indiquant l'absence de croissance dudit micro-organisme qui s'y trouve, et de la présence d'une variation importante de couleur dudit puits témoin de croissance positive par rapport à la couleur bleue initiale de ladite résazurine vers une couleur rouge pouvant être attribuée à la formation dudit produit de réduction consistant en résorufine provoquée par la croissance du micro-organisme qui s'y trouve ;
h2) d'indication d'un test infructueux si lesdits réceptacles ne sont pas qualifiés pour la lecture conformément à ladite étape h1) ; et
h3) d'indication d'un résultat d'essai si lesdits réceptacles sont qualifiés pour la lecture conformément à ladite étape i1) par détection de la présence ou de l'absence d'une variation importante de couleur dudit réceptacle d'essai à partir d'une couleur bleue initiale de la résazurine vers la couleur rouge de la résorufine, ledit résultat d'essai étant positif (croissance de l'organisme) si ledit décalage de couleur est présent et ledit résultat d'essai étant négatif (aucune croissance de l'organisme) si ladite variation de couleur est absente.

12. Procédé suivant la revendication 3, dans lequel ladite étape j1) est mise en oeuvre
k1) en introduisant dans ledit réceptacle d'essai un volume, ajusté préalablement, de liquide renfermant ladite concentration choisie préalablement dudit produit antimicrobien, ladite concentration ajustée préalablement de ladite résasurine, ladite concentration ajustée préalablement dudit milieu de croissance, ledit tampon choisi préalablement et ledit stabilisant redox choisi préalablement ; et
k2) en séchant ledit volume de liquide pour capturer ledit produit antimicrobien, ladite résazurine, ledit milieu de croissance, ledit tampon et ledit stabilisant redox sous forme de constituants solides secs,
et ladite étape j2) est mise en oeuvre
11) en introduisant dans ledit réceptacle d'essai un volume de liquide comprenant ladite concentration, ajustée préalablement, dudit micro-organisme pour réhydrater lesdits constituants solides secs.

13. Procédé suivant la revendication 5, dans lequel ledit milieu de support comprend un disque de papier absorbant et lesdits constituants solides secs sont capturés dans ledit disque.

14. Procédé suivant la revendication 5, dans lequel ledit milieu de support comprend au moins des premier et second disques de papier absorbant, ledit premier disque renfermant ladite résazurine, ledit second disque renfermant ledit milieu de croissance, et ledit tampon et ledit stabilisant redox étant introduits dans un desdits premier et second disques.

15. Procédé suivant la revendication 5, dans lequel ladite étape (g) comprend la mise en incubation desdits réceptacles conjointement pendant un temps d'incubation prédéterminé associé à un protocole de lecture de lumière visible ; et
ladite étape (h) comprend les étapes
h1) de qualification desdits réceptacles pour la lecture sur la base de la détection de l'absence d'une variation discernable dans la couleur bleue initiale pouvant être attribuée à la résazurine dans ledit réceptacle témoin de croissance négative, indiquant l'absence de croissance dudit micro-organisme qui s'y trouve, et de la présence d'une variation importante de couleur dudit puits témoin de croissance positive à partir de la couleur bleue initiale de ladite résazurine vers une couleur rouge attribuable à la formation dudit produit de réduction consistant en résorufine provoquée par la croissance du micro-organisme qui s'y trouve ;
h2) d'indication d'un test infructueux si lesdits réceptacles ne sont pas qualifiés pour la lecture conformément à ladite étape h1) ; et
h3) d'indication d'un résultat d'essai si lesdits réceptacles sont qualifiés pour la lecture en fonction de ladite étape I1) par détection de la présence ou de l'absence d'une variation importante de couleur dudit réceptacle d'essai à partir d'une couleur bleue initiale de la résazurine vers la couleur rouge de la résorufine, ledit résultat d'essai étant positif (croissance de l'organisme) si ledit décalage de couleur est présent et ledit résultat d'essai étant négatif (aucune croissance de l'organisme) si ladite variation de couleur est absente.

16. Appareil pour tester la sensibilité qualitative d'un micro-organisme à une inhibition de croissance par un produit antimicrobien au moyen d'un protocole d'essai de sensibilité qualitative conçu préalablement faisant intervenir des première et seconde quantités dudit produit anti-microbien, comprenant
un panneau d'essai définissant un puits témoin de croissance négative, un puits témoin de croissance positive et une paire de puits d'essai ;
un module d'essai porté par chacun des puits consistant en ledit puits témoin de croissance négative, ledit puits témoin de croissance positive et ladite paire de puits d'essai, chacun desdits modules d'essai comprenant un volume solide sec d'une sous-catégorie des constituants d'une série d'agents chimiques d'essai comprenant une quantité de ladite résazurine et une quantité de milieu de croissance pour les micro-organismes, et un stabilisant redox, choisi préalablement, **caractérisé par** une diminution substantielle de la réduction de la résazurine par ledit milieu de croissance, chacun desdits modules d'essai dans ladite paire de puits d'essai comprenant en outre un volume solide sec d'une desdites première et seconde quantités dudit produit antimicrobien ;
ledit module d'essai dans ledit puits témoin de croissance négative étant apte à la réhydratation par un volume de liquide stérile contenant l'ensemble des constituants de ladite série d'agents chimiques d'essai non présents dans ladite sous-catégorie ;
lesdits modules d'essai dans chacun des puits consistant en ledit puits témoin de croissance positive et ladite paire de puits d'essai étant aptes à la réhydratation par un volume d'inoculum liquide contenant tous le constituants de ladite série d'agents chimiques d'essai non présents dans ladite sous-catégorie conjointement avec une concentration ajustée préalablement dudit micro-organisme ;
ledit volume de liquide stérile et ledit volume d'inoculum liquide renfermant la même quantité en volume choisie préalablement, ladite quantité de ladite résazurine étant choisie préalablement par rapport à ladite quantité en volume choisie préalablement pour engendrer une concentration choisie préalablement de ladite résazurine dans chacun desdits puits dans un intervalle **caractérisé par** une faible toxicité vis-à-vis de la croissance du micro-organisme et une sensibilité importante à la réduction en résorufine par les produits métaboliques de croissance du micro-organisme, et ladite quantité dudit milieu de croissance étant choisie préalablement pour engendrer une concentration choisie préalablement d'un milieu de croissance dans chacun desdits puits ;
ledit panneau d'essai et lesdits modules d'essai réhydratés portés par ce panneau étant aptes à la mise en incubation pendant un temps d'incubation associé à un protocole de lecture choisi préalablement comprenant un des protocoles consistant en un protocole de lecture de lumière visible et un protocole de lecture d'excitation fluorescente, et à être ensuite soumis à une lecture conformément audit protocole de lecture choisi préalablement pour déterminer la présence ou l'absence de croissance dudit micro-organisme dans lesdits puits d'essai.

17. Appareil pour tester la sensibilité quantitative d'un micro-organisme à l'inhibition de croissance par un produit antimicrobien au moyen d'un protocole d'essai de sensibilité quantitative conçu préalablement impliquant N quantités différentes dudit produit antimicrobien, N étant supérieur à 2, comprenant
un panneau d'essai définissant un puits témoin de croissance négative, un puits témoin de croissance positive et N puits d'essai,
un module d'essai porté par chacun des puits consistant en ledit puits témoin de croissance négative, ledit puits témoin de croissance positive et lesdits N puits d'essai, chacun desdits modules d'essai comprenant un volume solide sec d'une sous-catégorie des constituants d'une série d'agents chimiques d'essai comprenant une quantité de ladite résazurine et une quantité dudit milieu de croissance pour les micro-organismes et un stabilisant redox, choisi préalablement, **caractérisé par** une diminution substantielle de la réduction de la résazurine par ledit milieu de croissance, chacun desdits modules d'essai dans lesdits N puits d'essai comprenant en outre un volume solide sec d'une quantité différente dudit produit antimicrobien choisie préalablement conformément audit protocole d'essai de sensibilité quantitative ;
ledit module d'essai dans ledit puits témoin de croissance négative étant apte à la réhydratation par un volume de liquide stérile contenant l'ensemble des constituants de ladite série d'agents chimiques d'essai non présents dans ladite sous-catégorie ;
lesdits modules d'essai dans chacun des puits consistant en ledit puits témoin de croissance positive et lesdits N puits d'essais étant aptes à la réhydratation par un volume d'inoculum liquide contenant l'ensemble des constituants de ladite série d'agents chimiques d'essai non présents dans ladite sous-catégorie conjointement avec une concentration ajustée préalablement dudit micro-organisme ;
ledit volume de liquide stérile et ledit volume d'inoculum liquide renfermant la même quantité en volume choisie préalablement, ladite quantité de ladite résazurine étant choisie préalablement par rapport à ladite quantité en volume choisie préalablement pour engendrer une concentration choisie préalablement de ladite résazurine dans chacun desdits puits dans un intervalle **caractérisé par** une faible toxicité vis-à-vis de la croissance du micro-organisme et une sensibilité importante à la réduction en résorufine par les produits métaboliques de croissance du micro-organisme, et ladite quantité de milieu de croissance étant choisie préalablement pour engendrer une concentration choisie préalablement de milieu de croissance dans chacun desdits puits ;
ledit panneau d'essai et lesdits modules d'essai réhydratés portés par ce panneau étant aptes à la mise en incubation pendant un temps d'incubation associé à un protocole de lecture choisi préalablement comprenant un des protocoles consistant en un protocole de lecture de lumière visible et un protocole de lecture d'excitation de fluorescence, et à être ensuite soumis à une lecture conformément audit protocole de lecture choisi préalablement pour déterminer la présence ou l'absence de croissance dudit micro-organisme dans lesdits puits d'essai.

18. Appareil suivant la revendication 16 ou la revendication 17, dans lequel chacun des modules d'essai comprend un milieu de support porté par un puits associé et chacun desdits modules d'essai porte la sous-catégorie des constituants de ladite série d'agents chimiques d'essai.

19. Appareil suivant la revendication 18, dans lequel ledit milieu de support comprend un disque de papier absorbant et chacun desdits disques de papier absorbant dans les puits d'essai porte par impression une désignation, pouvant être lue visuellement, du nom et de ladite concentration dudit produit antimicrobien que renferme ce disque.

20. Appareil suivant la revendication 18, dans lequel ledit milieu de support comprend au moins des premier et second disques de papier absorbant, ledit premier disque portant ladite résazurine et ledit second disque portant ledit milieu de croissance, un desdits premier et second disques de papier de chacun des puits d'essai portant ladite qualité associée du produit antimicrobien et portant par impression une désignation, pouvant être lue visuellement, du nom et de ladite concentration dudit produit antimicrobien présent dans ce disque.

21. Kit pour tester la sensibilité qualitative d'un micro-organisme à l'inhibition de croissance par un produit antimicrobien au moyen d'un protocole d'essai de sensibilité qualitative conçu préalablement faisant intervenir des première et seconde quantités dudit produit antimicrobien, comprenant :
un panneau d'essai définissant un puits témoin de croissance négative, un puits témoin de croissance positive et une paire de puits d'essai ;
un module d'essai porté par chacun des puits consistant en ledit puits témoin de croissance négative, ledit puits témoin de croissance positive et ladite paire de puits d'essai, chacun desdits modules d'essai comprenant un volume solide sec d'une sous-catégorie des constituants d'une série d'agents chimiques d'essai comprenant une quantité de ladite résazurine et une quantité de milieu de croissance pour les micro-organismes et un stabilisant redox, choisi préalablement, **caractérisé par** une diminution substantielle de la réduction de la résazurine par ledit milieu de croissance, chacun desdits modules d'essai dans ladite paire de puits d'essai comprenant en outre un volume solide sec d'une desdites première et seconde quantités dudit produit antimicrobien ;
un récipient de liquide réhydratant contenant l'ensemble des constituants de ladite série d'agents chimiques d'essai non présents dans ladite sous-catégorie ;
un moyen de stockage d'inoculum pour stocker un volume dudit liquide réhydratant auquel a été ajouté ledit micro-organisme pour former un inoculum liquide renfermant une concentration ajustée préalablement du micro-organisme ;
un moyen d'inoculation comprenant un moyen pour introduire une quantité ajustée préalablement dudit liquide réhydratant dans ledit puits témoin de croissance négative, un moyen pour introduire une quantité ajustée préalablement dudit inoculum liquide dans ledit puits témoin de croissance positive, et ladite paire de puits d'essai pour réhydrater les agents chimiques d'essai dans lesdits modules d'essai qui s'y trouvent ;
ladite quantité de ladite résazurine étant choisie préalablement par rapport à ladite quantité ajustée préalablement pour engendrer une concentration choisie préalablement de ladite résazurine dans chacun desdits puits dans un intervalle **caractérisé par** une faible toxicité vis-à-vis de la croissance du micro-organisme et une sensibilité importante à la réduction en résorufine par les produits métaboliques de croissance du micro-organisme, et ladite quantité de milieu de croissance étant choisie préalablement pour engendrer une concentration choisie préalablement de milieu de croissance standard dans chacun desdits puits ;
ledit panneau d'essai et lesdits modules d'essai réhydratés qui s'y trouvent étant aptes à être mis en incubation pendant un temps d'incubation associé à un protocole de lecture choisi préalablement comprenant un protocole consistant en un protocole de lecture de lumière visible et un protocole de lecture d'excitation de fluorescence, et ensuite à être soumis à une lecture conformément audit protocole de lecture choisi préalablement pour déterminer la présence ou l'absence de croissance dudit micro-organisme dans lesdits puits d'essai.

22. Kit pour tester la sensibilité quantitative d'un micro-organisme à l'inhibition de croissance par un produit antimicrobien au moyen d'un protocole d'essai de sensibilité quantitative conçu préalablement impliquant N quantités différentes dudit produit antimicrobien, N étant supérieur à 2, comprenant
un panneau d'essai définissant un puits témoin de croissance négative, un puits témoin de croissance positive et N puits d'essai ;
un module d'essai porté par chacun des puits consistant en ledit puits témoin de croissance négative, ledit puits témoin de croissance positive et lesdits N puits d'essai, chacun desdits modules d'essai comprenant un volume solide sec d'une sous-catégorie des constituants d'une série d'agents chimiques d'essai comprenant une quantité de résazurine et une quantité de milieu de croissance pour les micro-organismes et un stabilisant redox, choisi préalablement, **caractérisé par** une diminution substantielle de la réduction de la résazurine par ledit milieu de croissance, chacun desdits modules d'essai dans lesdits N puits d'essai comprenant en outre un volume solide sec d'une desdites N quantités différentes dudit produit antimicrobien ;
un récipient de liquide réhydratant contenant l'ensemble des constituants de ladite série d'agents chimiques d'essai non présents dans ladite sous-catégorie ;
un moyen de stockage d'inoculum pour stocker un volume dudit liquide réhydratant auquel a été ajouté ledit micro-organisme pour former un inoculum liquide renfermant une concentration ajustée préalablement de micro-organismes ;
un moyen d'inoculation comprenant un moyen pour introduire une quantité ajustée préalablement dudit liquide réhydradant dans ledit puits témoin de croissance négative, un moyen pour introduire une quantité ajustée préalablement dudit inoculum liquide dans ledit puits témoin de croissance positive et chacun desdits N puits d'essai pour réhydrater les agents chimiques d'essai dans lesdits modules d'essai qui s'y trouvent ;
ladite quantité de ladite résazurine étant choisie préalablement par rapport à ladite quantité ajustée préalablement pour engendrer une concentration choisie préalablement de ladite résazurine dans chacun desdits puits dans un intervalle **caractérisé par** une faible toxicité vis-à-vis de la croissance du micro-organisme et une sensibilité importante à la réduction en résorufine par les produits métaboliques de croissance du micro-organisme, et ladite quantité de milieu de croissance étant choisie préalablement pour engendrer une concentration choisie préalablement de milieu de croissance standard dans chacun desdits puits ;
ledit panneau d'essai et lesdits modules d'essai réhydratés portés par ce panneau étant aptes à être mis en incubation pendant un temps d'incubation associé à un protocole de lecture choisi préalablement, comprenant un des protocoles consistant en un protocole de lecture de lumière visible et un protocole de lecture d'excitation de fluorescence, et ensuite être soumis à une lecture conformément audit protocole de lecture choisi préalablement pour déterminer la présence ou l'absence de croissance dudit micro-organisme dans lesdits puits d'essai.

23. Kit suivant la revendication 21 ou la revendication 22, dans lequel chacun desdits modules d'essai comprend un disque de papier absorbant et chacun desdits disques de papier absorbant dans lesdits puits d'essai porte par impression une désignation, pouvant être lue visuellement, du nom et de ladite concentration dudit produit antimicrobien qui y est stocké.

24. Kit suivant la revendication 21 ou la revendication 22, dans lequel chacun desdits modules d'essai comprend au moins des premier et second disques de papier absorbant, ledit premier disque portant ladite résazurine et ledit second disque portant ledit milieu de croissance, un desdits premier et second disques de papier dans chacun desdits puits d'essai portant ladite quantité associée dudit produit antimicrobien et portant par impression une désignation, pouvant être lue visuellement, du nom et de ladite concentration dudit produit antimicrobien qui y est stocké.

25. Kit suivant la revendication 21, comprenant un premier module d'essai et un second module d'essai comprenant un milieu de support destiné à porter sous une forme solide sèche ladite seconde quantité dudit produit antimicrobien et ledit groupe d'agents chimiques d'essai ; et
un moyen de distribution pour distribuer lesdits premier et second modules d'essai dans ladite paire de puits d'essai.

26. Kit suivant la revendication 25, comprenant en outre
une paire de modules d'essai de croissance comprenant chacun un milieu de support destiné à porter sous une forme solide sèche ledit groupe d'agents chimiques d'essai ; et
un récipient dudit liquide réhydratant ;
ledit milieu de distribution comprenant un moyen pour distribuer un module de ladite paire de modules d'essai de croissance dans chacun des puits consistant en ledit puits témoin de croissance négative et ledit puits témoin de croissance positive ;
ledit moyen d'inoculation comprenant un moyen pour distribuer une quantité ajustée préalablement dudit liquide réhydratant dans ledit puits témoin de croissance négative et une quantité ajustée préalablement dudit inoculum dans ledit puits témoin de croissance positive.

27. Kit suivant la revendication 25, comprenant une multiplicité de panneaux d'essai pour tester des micro-organismes multiples et une multiplicité de chacun des modules consistant en ledit premier module d'essai et ledit second module d'essai ; et
ledit moyen de distribution comprenant des premier et second moyens de stockage pour stocker respectivement ladite multiplicité de premier et second modules d'essai, et un distributeur associé de manière fonctionnelle audit moyen de stockage pour distribuer simultanément un desdits premiers modules d'essai à partir dudit premier moyen de stockage dans un desdits puits d'essai et un desdits seconds modules d'essai à partir dudit second moyen de stockage dans l'autre desdits puits d'essai dans un panneau d'essai de ladite multiplicité de panneaux d'essai.

28. Kit suivant la revendication 25, comprenant une multiplicité de panneaux d'essai pour tester des micro-organismes multiples et une multiplicité de chacun des modules consistant en ledit premier module d'essai et ledit second module d'essai ;
ledit moyen de distribution comprenant un moyen de stockage pour stocker ladite multiplicité de premier et second modules d'essai de manière intercalée, et un distributeur associé de manière fonctionnelle audit moyen de stockage pour distribuer de manière alternée un premier module d'essai dans un desdits puits d'essai dans un desdits panneaux d'essai et un second module d'essai dans l'autre desdits puits d'essai dans ledit panneau desdits panneaux d'essai.

29. Kit suivant la revendication 23, dans lequel ladite série d'agents chimiques d'essai comprend en outre un tampon choisi préalablement pour l'ajustement du pH, et dans lequel tous les constituants de ladite série d'agents chimiques d'essai sont portés par ledit disque de papier absorbant, ledit inoculum liquide contenant seulement ledit micro-organisme.

30. Kit suivant la revendication 25, apte à tester la sensibilité qualitative dudit micro-organisme à l'inhibition de croissance par M produits antimicrobiens différents, dans lequel
ledit panneau définit M paires de puits d'essai, chaque paire étant associée à un desdits M produits antimicrobiens ;
M desdits premiers modules d'essai et M desdits seconds modules d'essai sont fournis, chacun étant associé à un desdits produits antimicrobiens ;
ledit moyen d'ensemencement comprend un moyen pour distribuer chacun desdits M premiers modules d'essai et chacun desdits M seconds modules d'essai dans les puits associés desdites M paires de puits d'essai ; et
ledit moyen d'inoculation distribue une quantité ajustée préalablement dudit inoculum liquide dans chaque puits desdites M paires de puits d'essai.

31. Kit suivant la revendication 30, dans lequel chacun desdits M premier et second modules d'essai comprend un disque de papier absorbant portant par impression une désignation, pouvant être lue visuellement, du nom et de ladite concentration dudit produit antimicrobien associé porté par ce disque.

32. Kit suivant la revendication 31, dans lequel ladite série d'agents chimiques d'essai comprend en outre un tampon choisi préalablement pour l'ajustement du pH et un stabilisant redox choisi préalablement pour diminuer la réduction de la résazurine par ledit milieu de croissance et ledit tampon au cours de l'incubation dudit panneau d'essai, et dans lequel l'ensemble des constituants de ladite série d'agents chimiques d'essai est porté par chacun desdits disques de papier absorbant, et ledit inoculum liquide contient seulement ledit micro-organisme.

33. Kit suivant la revendication 22, comprenant au moins N modules d'essai comprenant chacun un milieu de support destiné à porter sous une forme solide sèche une desdites N quantités différentes dudit produit antimicrobien et un groupe d'agents chimiques d'essai comprenant une sous-catégorie des constituants d'une série d'agents chimiques d'essai comprenant une quantité de ladite résazurine et une quantité d'un milieu de croissance pour des micro-organismes ; et
un moyen de distribution pour distribuer lesdits N modules d'essai dans lesdits N puits d'essai dans un ordre en fonction de la quantité dudit produit antimicrobien qui s'y trouve.

34. Kit suivant la revendication 33, comprenant une multiplicité de panneaux d'essai pour tester des micro-organismes multiples et une multiplicité de séries desdits N modules d'essai ;
ledit moyen de distribution comprenant un moyen de stockage pour stocker ladite multiplicité de séries de N modules d'essai et un moyen pour distribuer lesdits modules d'essai un par un à partir dudit moyen de stockage, lesdits N modules d'essai dans chacune de ladite multiplicité de séries étant organisés dans ledit moyen de stockage dans un ordre conçu préalablement en fonction de la quantité dudit produit antimicrobien qui s'y trouve de telle sorte que ledit moyen de distribution puisse agir en distribuant N modules d'essai dans ledit ordre dans lesdits N puits d'essai dans chacun desdits panneaux d'essai.

35. Kit suivant la revendication 23, dans lequel ladite série d'agents chimiques d'essai comprend en outre un tampon choisi préalablement pour l'ajustement du pH, et dans lequel l'ensemble des constituants de ladite série d'agents chimiques d'essai est porté par ledit disque de papier absorbant, l'inoculum liquide contenant seulement ledit micro-organisme.

36. Kit suivant la revendication 34, apte à tester la sensibilité quantitative dudit micro-organisme à l'inhibition de croissance par M produits antimicrobiens différents, dans lequel
ledit panneau d'essai définit M séries de N puits d'essai, chaque série étant associée à un desdits produits antimicrobiens ;
ledit moyen de distribution comprend M moyens distincts desdits moyens de stockage, chacun associé à un desdits M produits antimicrobiens et possédant des moyens associés pour distribuer N modules d'essai qui y sont stockés dans une série associée desdites M séries de N puits d'essai ;
ledit moyen d'inoculation distribuant ladite quantité ajustée préalablement dudit inoculum liquide dans chacun desdits N puits d'essai dans chacune desdites M séries de puits d'essai de manière séquentielle à raison d'une série à la fois.
